# EUROPEAN PATENT SPECIFICATION

(11) **EP 3 515 494 B1**
(45) Date of publication and mention of the grant of the patent: **25.02.2026**
(21) Application number: 17854126.4
(22) Date of filing: 26.09.2017
(51) Int. Cl.: A61K 39/395, C07K 16/28, A61P 37/00

(54) **REGULATORS OF B CELL-MEDIATED IMMUNOSUPPRESSION**
REGULATOREN DER B-ZELLVERMITTELTEN IMMUNSUPPRESSION
RÉGULATEURS DE L'IMMUNOSUPPRESSION MÉDIÉE PAR LYMPHOCYTES B

(30) Priority: 26.09.2016 US 201662399775 P
(43) Date of publication of application: 31.07.2019
(73) Proprietor: The Brigham and Women's Hospital, Inc., Boston, MA 02115 (US)
(72) Inventor: KUCHROO, Vijay, K., Newton, MA 02459 (US); XIAO, Sheng, Newton, MA 02458 (US)
(74) Representative: Kuttenkeuler, David
(86) International application number: PCT/US2017/053453
(87) International publication number: WO 2018/058111

(56) References cited:
- WO-A1-2015/103072
- WO-A1-2016/115274
- WO-A1-2017/185662
- WO-A2-2011/159877
- WO-A2-2016/090034
- CN-A- 106 939 050
- CN-A- 107 043 425
- US-A1- 2013 156 774
- JENNIFER HARTT MEYERS ET AL: "TIM-4 is the ligand for TIM-1, and the TIM-1-TIM-4 interaction regulates T cell proliferation", NATURE IMMUNOLOGY, vol. 6, no. 5, 27 March 2005 (2005-03-27), New York, pages 455 - 464, XP055225957, ISSN: 1529-2908, DOI: 10.1038/ni1185
- ZHANG QING ET AL: "Blockade of the checkpoint receptor TIGIT prevents NK cell exhaustion and elicits potent anti-tumor immunity", NATURE IMMUNOLOGY, NATURE PUBLISHING GROUP US, NEW YORK, vol. 19, no. 7, 18 June 2018 (2018-06-18), pages 723 - 732, XP036533618, ISSN: 1529-2908, [retrieved on 20180618], DOI: 10.1038/S41590-018-0132-0

## Description

### TECHNICAL FIELD

The technical field relates to compositions and methods for regulating immunosupression, particular to the invention defined by the appended set of claims.

### BACKGROUND

The immune system protects the body from foreign invaders and diseased cells; but immune disorders, particularly those associated with aberrant immune tolerance mechanisms, such as autoimmunity and cancers, can wreak havoc. According to the most recent data from the World Health Organization, ten million people around the world were diagnosed with cancer in 2000, and six million died from it. Moreover, statistics indicate that the cancer incidence rate is on the rise around the globe. In America, for example, projections suggest that fifty percent of those alive today will be diagnosed with some form of cancer at some point in their lives. Immune tolerance is also implicated in immune suppression that can be desirable, for example, in autoimmune diseases and in organ transplant situations, wherein an overactive immune response can cause great permanent damage to the afflicted individual and or donor organ. Autoimmune diseases are on the rise in the U.S. and around the world. In the U.S. alone, some fifty million are affected, and autoimmune disease is one of the top ten causes of death in women under the age of 65, is the second highest cause of chronic illness, and the top cause of morbidity in women.

### SUMMARY

The compositions and methods described herein are based, in part, on the discovery that regulatory B cells (Bregs) differentially express a specific set of coinhibitory molecules, including TIGIT, LAG-3, PD-1, CTLA4, and TIM-3. As shown herein, TIGIT (T-Cell Immunoreceptor with Ig and ITIM Domains) is required for the development and function of Bregs. Hosts with TIGIT deficiency specifically in B cells not only developed spontaneous paralytic disease with inflammation in brain, but also showed more severe experimental autoimmune encelphalomyelitis (EAE) without recovery after disease induction, thus indicating that TIGIT expression in Bregs is critical for their immunosuppressive function. The data described herein indicate that TIGIT is required for both Breg-mediated tolerance maintenance at the steady state, and inflammation restraint during autoimmune and inflammatory diseases. Accordingly, as described herein, compositions and methods targeting coinhibitory molecules, such as TIGIT, LAG-3, PD-1, CTLA4, and TIM-3, in B cells, can be used to provide novel therapeutic strategies for modulating immune suppression and treating diseases mediated or impacted by immune suppression mechanisms, such as autoimmune diseases and cancers.

Accordingly, provided herein, in some aspects, are methods of reducing B-cell-mediated immunosuppression comprising administering a therapeutically effective amount of an inhibitor of TIGIT, PD-1, TIM-3, LAG-3, or CTLA-4 activity or expression in B cells to a subject in need thereof.

In some embodiments of these aspects and all such aspects described herein, the inhibitor is specifically targeted to B cells.

In some embodiments of these aspects and all such aspects described herein, the inhibitor comprises a multispecific binding agent comprising a moiety that binds and inhibits the activity of TIGIT, PD-1, TIM-3, LAG-3, or CTLA-4, and a moiety that binds a B-cell-specific cell-surface polypeptide marker.

In some embodiments of these aspects and all such aspects described herein, the moiety that binds and inhibits the activity of TIGIT, PD-1, TIM-3, LAG-3, or CTLA-4 comprises an antigen-binding domain of an antibody that specifically binds TIGIT, PD-1, TIM-3, LAG-3, or CTLA-4, respectively.

In some embodiments of these aspects and all such aspects described herein, the moiety that binds a B-cell-specific cell-surface polypeptide marker comprises an antigen-binding domain of an antibody that specifically binds a B-cell-specific cell surface marker. In some embodiments of these aspects and all such aspects described herein, the B-cell-specific cell surface marker is selected from CD19, CD20, or CD22.

In some aspects, provided herein are methods of treating a disease or disorder involving inappropriate immunosuppression, the method comprising administering a therapeutically effective amount of an inhibitor of TIGIT, PD-1, TIM-3, LAG-3, or CTLA-4 activity or expression in B cells to a subject in need thereof.

In some embodiments of these aspects and all such aspects described herein, the inhibitor is specifically targeted to B cells.

In some embodiments of these aspects and all such aspects described herein, the inhibitor comprises a multispecific binding agent comprising a moiety that binds and inhibits the activity of TIGIT, PD-1, TIM-3, LAG-3, or CTLA-4, and a moiety that binds a B-cell-specific cell-surface polypeptide marker.

In some embodiments of these aspects and all such aspects described herein, the moiety that binds and inhibits the activity of TIGIT, PD-1, TIM-3, LAG-3, or CTLA-4 comprises an antigen-binding domain of an antibody that specifically binds TIGIT, PD-1, TIM-3, LAG-3, or CTLA-4, respectively.

In some embodiments of these aspects and all such aspects described herein, the moiety that binds a B-cell-specific cell-surface polypeptide marker comprises an antigen-binding domain of an antibody that specifically binds a B-cell-specific cell surface marker. In some embodiments of these aspects and all such aspects described herein, the B-cell-specific cell surface marker is selected from CD19, CD20, and CD22.

In some embodiments of these aspects and all such aspects described herein, the disease or disorder is selected from cancer and a chronic infection.

Provided herein, in some aspects, are methods of treating an autoimmune or inflammatory disease or disorder comprising administering therapeutically effective amount of an agent that increases the expression or activity of TIGIT, PD-1, TIM-3, LAG-3, or CTLA-4 in B cells to a subject in need thereof.

In some embodiments of these aspects and all such aspects described herein, the agent that increases the expression or activity of TIGIT in B cells comprises soluble TIM-4.

In some embodiments of these aspects and all such aspects described herein, the agent that increases the expression or activity of TIGIT in B cells is specifically targeted to B cells.

In some embodiments of these aspects and all such aspects described herein, the autoimmune or inflammatory disease or disorder is selected from the group consisting of multiple sclerosis, SLE, and rheumatoid arthritis.

In some aspects, provided herein are compositions comprising a multispecific binding agent comprising a moiety that binds and inhibits the activity of a B-cell regulator selected from TIGIT, PD-1, TIM-3, LAG-3, and CTLA-4, and a moiety that binds a B-cell-specific cell-surface polypeptide marker.

In some embodiments of these aspects and all such aspects described herein, the moiety that binds and inhibits the activity of the B-cell regulator comprises an antigen-binding domain of an antibody that specifically binds the B-cell regulator.

In some embodiments of these aspects and all such aspects described herein, the moiety that binds a B-cell-specific cell-surface polypeptide marker comprises an antigen-binding domain of an antibody that specifically binds a B-cell-specific cell surface marker.

In some embodiments of these aspects and all such aspects described herein, the antigen-binding domain is comprised by an scFV or a nanobody.

Provided herein, in some aspects, is a spontaneous animal model of multiple sclerosis, the model comprising a non-human mammal with a B cell-specific knockout of the TIM-1 gene.

Provided herein, in some aspects, is a spontaneous animal model of multiple sclerosis, the model comprising a non-human mammal with a B cell-specific knockout of the TIGIT gene.

In some embodiments of these aspects and all such aspects described herein, the mammal is a rodent.

In some embodiments of these aspects and all such aspects described herein, the rodent is a rat or a mouse.

In some embodiments of these aspects and all such aspects described herein, the mammal spontaneously develops paralysis.

### Definitions

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims. Definitions of common terms in immunology, and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737.

As used herein, "antibodies" or "antigen-binding fragments" thereof include monoclonal, human, humanized or chimeric antibodies, single chain antibodies, Fab fragments, F(ab') fragments, fragments produced by a Fab expression library, and/or antigen-binding fragments of any of the above. Antibodies also refer to immunoglobulin molecules and immunologically active portions of immunoglobulin molecules, *i.e.,* molecules that contain antigen or target binding sites or "antigen-binding fragments." The immunoglobulin molecules described herein can be of any type *(e.g.,* IgG, IgE, IgM, IgD, IgA and IgY), class *(e.g.,* IgG1, IgG2, IgG3, IgG4, IgA1 and IgA2) or subclass of immunoglobulin molecule, as is understood by one of skill in the art.

The terms "antibody fragment" or "antigen-binding fragment" include: (i) the Fab fragment, having V_{L}, C_{L}, V_{H} and C_{H}1 domains; (ii) the Fab' fragment, which is a Fab fragment having one or more cysteine residues at the C-terminus of the C_{H}1 domain; (iii) the Fd fragment having V_{H} and C_{H}1 domains; (iv) the Fd' fragment having V_{H} and C_{H}1 domains and one or more cysteine residues at the C-terminus of the CH1 domain; (v) the Fv fragment having the V_{L} and V_{H} domains of a single arm of an antibody; (vi) a dAb fragment (Ward et al., Nature 341, 544-546 (1989)) which consists of a V_{H} domain or a V_{L} domain ; (vii) isolated CDR regions; (viii) F(ab')₂ fragments, a bivalent fragment including two Fab' fragments linked by a disulphide bridge at the hinge region; (ix) single chain antibody molecules *(e.g.* single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (V_{H}) connected to a light chain variable domain (V_{L}) in the same polypeptide chain (see, *e.g.,* EP 404,097; WO 93/11161; and Hollinger et al., Proc. Natl. Acad. Sci. USA, 90:6444-6448 (1993)); (xi) "linear antibodies" comprising a pair of tandem Fd segments (V_{H}-C_{H}1-V_{H}-C_{H}1) which, together with complementary light chain polypeptides, form a pair of antigen binding regions (Zapata et al. Protein Eng. 8(10):1057-1062 (1995); and U.S. Pat. No. 5,641,870); and modified versions of any of the foregoing (e.g., modified by the covalent attachment of polyalkylene glycol (e.g., polyethylene glycol, polypropylene glycol, polybutylene glycol) or other suitable polymer).

The term "epitope" includes any polypeptide determinant capable of specific binding to an immunoglobulin or T-cell receptor. In certain embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. An epitope is a region of an antigen that is bound by a binding protein. An epitope may be determined by obtaining an X-ray crystal structure of an antibody:antigen complex and determining which residues on the antigen are within a specified distance of residues on the antibody of interest, wherein the specified distance is, 5Å or less, e.g., 5Å, 4Å, 3Å, 2Å, 1Å or any distance in between. In some embodiments, an "epitope" can be formed on a polypeptide both from contiguous amino acids, or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5, about 9, or about 8-10 amino acids in a unique spatial conformation. An "epitope" includes the unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation. The terms "antigenic determinant" and "epitope" can also be used interchangeably herein. In certain embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. In some embodiments, an epitope comprises of 8 or more contiguous or non-contiguous amino acid residues in the TIGIT, PD-1, TIM-3, LAG-3, or CTLA-4 sequence in which at least 50%, 70% or 85% of the residues are within the specified distance of the antibody or binding protein in the X-ray crystal structure.

The terms "specificity" or "specific for" refers to the number of different types of antigens or antigenic determinants to which a binding protein, antibody or antibody fragment, or antigen-binding portion thereof thereof as described herein can bind. The specificity of a binding protein, antibody or antibody fragment, or antigen-binding portion thereof thereof can be determined based on affinity and/or avidity. The affinity, represented by the equilibrium constant for the dissociation (K_{D}) of an antigen with an antigen-binding protein, is a measure of the binding strength between an antigenic determinant and an antigen-binding site on the antigen-binding protein, such as a binding protein, antibody or antibody fragment, or antigen-binding portion thereof thereof: the lesser the value of the K_{D}, the stronger the binding strength between an antigenic determinant and the antigen-binding molecule. Alternatively, the affinity can also be expressed as the affinity constant (K_{A}), which is 1/ K_{D}). As will be clear to the skilled person, affinity can be determined in a manner known per se, depending on the specific antigen of interest. Accordingly, a binding protein, antibody or antibody fragment, or antigen-binding portion thereof thereof as defined herein is said to be "specific for" a first target or antigen compared to a second target or antigen when it binds to the first antigen with an affinity (as described above, and suitably expressed, for example as a K_{D} value) that is at least 10 times, such as at least 100 times, and preferably at least 1000 times, and up to 10000 times or more better than the affinity with which said amino acid sequence or polypeptide binds to another target or polypeptide.

Accordingly, as used herein, "selectively binds" or "specifically binds" or "specific binding" in reference to the interaction of an antibody, or antibody fragment thereof, or a binding protein described herein, means that the interaction is dependent upon the presence of a particular structure (e.g., an antigenic determinant or epitope or target) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody. In certain embodiments, a binding protein or antibody or antigen-binding fragment thereof that specifically binds to an antigen binds to that antigen with a K_{D} greater than 10⁻⁶ M, 10⁻⁷ M, 10⁻⁸ M, 10⁻⁹ M, 10⁻¹⁰ M, 10⁻¹¹ M, 10⁻¹² M, 10⁻¹³ M, 10⁻¹⁴ M. In other embodiments, a binding protein or antibody or antigen binding fragment thereof that specifically binds to an antigen binds to that antigen with a K_{D} between 10⁻⁶ and 10⁻⁷M, 10⁻⁶ and 10⁻⁸ M, 10⁻⁶ and 10⁻⁹ M, 10⁻⁶ and 10⁻¹⁰ M, 10⁻⁶ and 10⁻¹¹ M, 10⁻⁶ and 10⁻¹²M, 10⁻⁶ and 10⁻¹³ M, 10⁻⁶ and 10⁻¹⁴ M, 10⁻⁹ and 10⁻¹⁰ M, 10⁻⁹ and 10⁻¹¹ M, 10⁻⁹ and 10⁻¹² M, 10⁻⁹ and 10⁻¹³ M, 10⁻⁹ and 10⁻¹⁴ M. In some embodiments, a binding protein or antibody or antigen-binding fragment thereof binds to an epitope, with a K_{D} 10⁻⁵ M (10000 nM) or less, *e.g.,* 10⁻⁶ M, 10⁻⁷ M, 10⁻⁸ M, 10⁻⁹ M, 10⁻¹⁰ M, 10⁻¹¹ M, 10⁻¹² M, or less. Specific binding can be influenced by, for example, the affinity and avidity of the polypeptide agent and the concentration of polypeptide agent. The person of ordinary skill in the art can determine appropriate conditions under which the polypeptide agents described herein selectively bind the targets using any suitable methods, such as titration of a polypeptide agent in a suitable cell binding assay. In certain embodiments, a binding protein or antibody or antigen-binding fragment thereof is said to "specifically bind" an antigen when it preferentially recognizes its target antigen in a complex mixture of proteins and/or macromolecules. Binding proteins, antibodies or antigen-binding fragments that bind to the same or similar epitopes will likely cross-compete (one prevents the binding or modulating effect of the other). Cross-competition, however, can occur even without epitope overlap, e.g., if epitopes are adjacent in three-dimensional space and/or due to steric hindrance.

Avidity is the measure of the strength of binding between an antigen-binding molecule (such as a binding protein, antibody or antibody fragment, or antigen-binding portion thereof thereof described herein) and the pertinent antigen. Avidity is related to both the affinity between an antigenic determinant and its antigen binding site on the antigen-binding molecule, and the number of pertinent binding sites present on the antigen-binding molecule. Typically, antigen-binding proteins (such as a binding protein, antibody or antibody fragment, or antigen-binding portion thereof thereof described herein) will bind to their cognate or specific antigen with a dissociation constant (K_{D} of 10⁻⁵ to 10⁻¹² moles/liter or less, and preferably 10⁻⁷ to 10⁻¹² moles/liter or less and more preferably 10⁻⁸ to 10⁻¹² moles/liter *(i.e.,* with an association constant (K_{A}) of 10⁵ to 10¹² liter/moles or more, and preferably 10⁷ to 10¹² liter/moles or more and more preferably 10⁸ to 10¹² liter/moles). Any K_{D} value greater than 10⁻⁴ mol/liter (or any K_{A} value lower than 10⁴ M⁻¹) is generally considered to indicate non-specific binding. The K_{D} for biological interactions which are considered meaningful (e.g., specific) are typically in the range of 10⁻¹⁰ M (0.1 nM) to 10⁻⁵ M (10000 nM). The stronger an interaction is, the lower is its K_{D}. Preferably, a binding site on a binding protein, antibody or antibody fragment, or antigen-binding portion thereof thereof described herein will bind to the desired antigen with an affinity less than 500 nM, preferably less than 200 nM, more preferably less than 10 nM, such as less than 500 pM. Specific binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as other techniques as mentioned herein.

As used herein, an "antigen-binding fragment" refers to a protein fragment that comprises at least an antigen binding site of the intact antibody and thus retains the ability to bind a given target antigen or epitope. Non-limiting examples of antibody fragments encompassed by the term antigen-binding fragment include: (i) the Fab fragment, having V_{L}, C_{L}, V_{H} and C_{H}1 domains; (ii) the Fab' fragment, which is a Fab fragment having one or more cysteine residues at the C-terminus of the C_{H}1 domain; (iii) the Fd fragment having V_{H} and C_{H}1 domains; (iv) the Fd' fragment having V_{H} and C_{H}1 domains and one or more cysteine residues at the C-terminus of the CH1 domain; (v) the Fv fragment having the V_{L} and V_{H} domains of a single arm of an antibody; (vi) the dAb fragment (Ward et al., Nature 341, 544-546 (1989)) which consists of a V_{H} domain; (vii) isolated CDR regions; (viii) F(ab')₂ fragments, a bivalent fragment including two Fab' fragments linked by a disulphide bridge at the hinge region; (ix) single chain antibody molecules *(e.g.,* single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (V_{H}) connected to a light chain variable domain (V_{L}) in the same polypeptide chain (see, *e.g.,* EP 404,097; WO 93/11161; and Hollinger et al., Proc. Natl. Acad. Sci. USA, 90:6444-6448 (1993)); (xi) "linear antibodies" comprising a pair of tandem Fd segments (V_{H}-C_{H}1-V_{H}-C_{H}1) which, together with complementary light chain polypeptides, form a pair of antigen binding regions (Zapata et al. Protein Eng. 8(10):1057-1062 (1995); and U.S. Pat. No. 5,641,870).

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the V_{H}-V_{L} dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

The "Fab" fragment contains a variable and constant domain of the light chain and a variable domain and the first constant domain (C_{H}1) of the heavy chain. F(ab') ₂ antibody fragments comprise a pair of Fab fragments which are generally covalently linked near their carboxy termini by hinge cysteines between them. Other chemical couplings of antibody fragments are also known in the art.

"Single-chain Fv" or "scFv" antibody fragments comprise the V_{H} and V_{L} domains of antibody, wherein these domains are present in a single polypeptide chain. Generally the Fv polypeptide further comprises a polypeptide linker between the V_{H} and V_{L} domains, which enables the scFv to form the desired structure for antigen binding. For a review of scFv, see Pluckthun in The Pharmacology of Monoclonal Antibodies, Vol 113, Rosenburg and Moore eds. Springer-Verlag, New York, pp. 269-315 (1994).

The term "monoclonal antibody" or "mAb" as used herein refers to an antibody obtained from a population of substantially homogeneous antibodies, *i.e.,* the individual antibodies comprising the population are identical except for possible naturally occurring mutations that can be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring production of the antibody by any particular method. For example, the monoclonal antibodies to be used in accordance with the invention can be made by the hybridoma method first described by Kohler et al., Nature 256:495 (1975), or can be made by recombinant DNA methods (see, *e.g.,* U.S. Pat. No. 4,816,567). The "monoclonal antibodies" can also be isolated from phage antibody libraries using the techniques described in Clackson et al., Nature 352:624-628 (1991) or Marks et al., J. Mol. Biol. 222:581-597 (1991), for example. A monoclonal antibody can be of any species, including, but not limited to, mouse, rat, goat, rabbit, and human monoclonal antibodies. Various methods for making monoclonal antibodies specific for TIGIT, PD-1, TIM-3, LAG-3, or CTLA-4 as described herein are available in the art. For example, the monoclonal antibodies can be made using the hybridoma method first described by Kohler et al., Nature, 256:495 (1975), or by recombinant DNA methods (U.S. Pat. No. 4,816,567). "Monoclonal antibodies" can also be isolated from or produced using phage antibody libraries using the techniques originally described in Clackson et al., Nature 352:624-628 (1991), Marks et al., J. Mol. Biol. 222:581-597 (1991), McCafferty et al., Nature, 348:552-554 (1990), Marks et al., Bio/Technology, 10:779-783 (1992)), Waterhouse et al., Nuc. Acids. Res., 21:2265-2266 (1993), and techniques known to those of ordinary skill in the art.

The term "human antibody" includes antibodies having variable and constant regions derived from human germline immunoglobulin sequences. The human antibodies of the disclosure may include amino acid residues not encoded by human germline immunoglobulin sequences (e.g., mutations introduced by random or site-specific mutagenesis in vitro or by somatic mutation in vivo), for example in the CDRs and in particular CDR3. However, the term "human antibody" does not include antibodies in which CDR sequences derived from the germline of another mammalian species, such as a mouse, have been grafted onto human framework sequences.

The term "recombinant human antibody" includes all human antibodies that are prepared, expressed, created or isolated by recombinant means, such as antibodies expressed using a recombinant expression vector transfected into a host cell, antibodies isolated from a recombinant, combinatorial human antibody library, antibodies isolated from an animal (e.g., a mouse) that is transgenic for human immunoglobulin genes, or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

The term "chimeric antibody" refers to antibodies that comprise heavy and light chain variable domain sequences from one species and constant region sequences from another species, such as antibodies having murine heavy and light chain variable domains linked to human constant regions.

The term "CDR-grafted antibody" refers to antibodies that comprise heavy and light chain variable domain sequences from one species but in which the sequences of one or more of the CDR regions of VH and/or VL are replaced with CDR sequences of another species, such as antibodies having murine heavy and light chain variable domains in which one or more of the murine CDRs (e.g., CDR3) has been replaced with human CDR sequences.

The term "CDR" refers to the complementarity determining region within antibody variable sequences. There are three CDRs in each of the variable domains of the heavy chain and the light chain, which are designated CDR1, CDR2 and CDR3, for each of the variable domains. The term "CDR set" as used herein refers to a group of three CDRs that occur in a single variable domain capable of binding the antigen. The exact boundaries of these CDRs have been defined differently according to different systems. The system described by Kabat (Kabat et al., Sequences of Proteins of Immunological Interest, National Institutes of Health, Bethesda, Md. (1987) and (1991)) not only provides an unambiguous residue numbering system applicable to any variable domain of an antibody, but also provides precise residue boundaries defining the three CDRs. These CDRs may be referred to as Kabat CDRs. Chothia and coworkers (Chothia et al. (1987) J. Mol. Biol. 196: 901-917; and Chothia et al. (1989) Nature 342: 877-883) found that certain sub-portions within Kabat CDRs adopt nearly identical peptide backbone conformations, despite having great diversity at the level of amino acid sequence. These sub-portions were designated as L1, L2, and L3 or H1, H2, and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan et al. ((1995) FASEB J. 9:133-139) and MacCallum et al. ((1996) J. Mol. Biol. 262(5):732-745). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although exemplary embodiments use Kabat or Chothia defined CDRs.

The terms "Kabat numbering", "Kabat definitions", and "Kabat labeling" are used interchangeably herein. These terms, which are recognized in the art, refer to a system of numbering amino acid residues which are more variable (i.e., hypervariable) than other amino acid residues in the heavy and light chain variable domains of an antibody, or an antigen binding portion thereof (Kabat et al. (1971) Ann. NY Acad. Sci. 190:382-391; and Kabat et al. (1991) Sequences of Proteins of Immunological Interest, Fifth Edition, U.S. Department of Health and Human Services, NIH Publication No. 91-3242, which is also available on the world wide web. The "EU index as in Kabat" refers to the residue numbering of the human IgG1 EU antibody. As used herein, "Kabat sequence numbering" refers to numbering of the sequence encoding a variable region according to the EU index as in Kabat. In some embodiments, IMGT (INTERNATIONAL IMMUNOGENETICS INFORMATION SYSTEM) numbering of variable regions can also be used, which is the numbering of the residues in an immunoglobulin variable heavy or light chain according to the methods of the IIMGT, as described in Lefranc, M.-P., "The IMGT unique numbering for immunoglobulins, T cell Receptors and Ig-like domains", The Immunologist, 7, 132-136 (1999). As used herein, "IMGT sequence numbering" refers to numbering of the sequence encoding a variable region according to the IMGT. For the heavy chain variable domain, the hypervariable region ranges from amino acid positions 31 to 35 for CDR1, amino acid positions 50 to 65 for CDR2, and amino acid positions 95 to 102 for CDR3. For the light chain variable domain, the hypervariable region ranges from amino acid positions 24 to 34 for CDR1, amino acid positions 50 to 56 for CDR2, and amino acid positions 89 to 97 for CDR3.

As used herein, the term "canonical" residue refers to a residue in a CDR or framework that defines a particular canonical CDR structure as defined by Chothia et al. ((1987) J. Mol. Biol. 196: 901-917); and Chothia et al. ((1992) J. Mol. Biol. 227: 799-817)). According to Chothia et al., critical portions of the CDRs of many antibodies have nearly identical peptide backbone confirmations despite great diversity at the level of amino acid sequence. Each canonical structure specifies primarily a set of peptide backbone torsion angles for a contiguous segment of amino acid residues forming a loop.

As used herein, "antibody variable domain" refers to the portions of the light and heavy chains of antibody molecules that include amino acid sequences of Complementarity Determining Regions (CDRs; *i.e.,* CDR1, CDR2, and CDR3), and Framework Regions (FRs). Each heavy chain is composed of a variable region of the heavy chain (V_{H} refers to the variable domain of the heavy chain) and a constant region of said heavy chain. The heavy chain constant region consists of three domains CH1, CH2 and CH3. Each light chain is composed of a variable region of said light chain (V_{L} refers to the variable domain of the light chain) and a constant region of the light chain. The light chain constant region consists of a CL domain. The VH and VL regions can be further divided into hypervariable regions referred to as complementarity-determining regions (CDRs) and interspersed with conserved regions referred to as framework regions (FR). Each VH and VL region thus consists of three CDRs and four FRs that are arranged from the N terminus to the C terminus in the following order: FR1, CDR1, FR2, CDR2, FR3, CDR3, FR4. This structure is well known to those skilled in the art. According to the methods used herein, the amino acid positions assigned to CDRs and FRs can be defined according to Kabat (Sequences of Proteins of Immunological Interest (National Institutes of Health, Bethesda, Md., 1987 and 1991)). Amino acid numbering of antibodies or antigen binding fragments is also according to that of Kabat.

As used herein, the term "Complementarity Determining Regions" ("CDRs"), *i.e.,* CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region can comprise amino acid residues from a "complementarity determining region" as defined by Kabat *(i.e.,* about residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, Md. (1991)) and/or those residues from a "hypervariable loop" *(i.e.,* about residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). In some instances, a complementarity determining region can include amino acids from both a CDR region defined according to Kabat and a hypervariable loop.

As used herein, the term "CDR" refers to the complementarity determining region within antibody variable sequences. There are three CDRs in each of the variable regions of the heavy chain and of the light chain, which are designated CDR 1, CDR2 and CDR3, for each of the variable regions. The term "CDR set" as used herein refers to a group of three CDRs that occur in a single variable region capable of binding the antigen. The exact boundaries of these CDRs have been defined differently according to different systems. The system described by Kabat (Kabat et al, Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Each complementarity determining region may comprise amino acid residues from a "complementarity determining region" as defined by Kabat (*i.e.* about residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, Md. (1991)) and/or those residues from a "hypervariable loop" (*i.e.* about residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). In some instances, a complementarity determining region can include amino acids from both a CDR region defined according to Kabat and a hypervariable loop. For example, the CDRH1 of the human heavy chain of antibody 4D5 includes amino acids 26 to 35. Chothia and coworkers (Chothia & Lesk, J. Mol. Biol, 196:901-917 (1987) and Chothia et al., Nature 342:877-883 (-1989)) found that certain sub-portions within Kabat CDRs adopt nearly identical peptide backbone conformations, in spite of great diversity at the level of amino acid sequence. These sub-portions were designated as L1, L2 and L3 or H1, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB ). 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although preferred embodiments use Kabat or Chothia defined CDRs. As used herein, "antibody variable domain" refers to the portions of the light and heavy chains of antibody molecules that include amino acid sequences of Complementarity Determining Regions (CDRs; ie., CDR1, CDR2, and CDR3), and Framework Regions (FRs). V_{H} refers to the variable domain of the heavy chain. V_{L} refers to the variable domain of the light chain. According to the methods used in this invention, the amino acid positions assigned to CDRs and FRs may be defined according to Kabat (Sequences of Proteins of Immunological Interest (National Institutes of Health, Bethesda, Md., 1987 and 1991)). Amino acid numbering of antibodies or antigen binding fragments is also according to that of Kabat. CDRs can also be described as comprising amino acid residues from a "complementarity determining region" as defined by the IMGT, in some embodiments.

The term "multivalent binding protein" denotes a binding protein comprising two or more antigen binding sites or domains. A multivalent binding protein may be engineered to have two or more antigen binding sites or domains, and is generally not a naturally occurring antibody. The term "multispecific binding protein" refers to a binding protein capable of binding two or more related or unrelated targets.

Similarily, unless indicated otherwise, the expression "multivalent antibody" is used throughout this specification to denote an antibody comprising two or more antigen binding sites. For example, the multivalent antibody is engineered to have the three or more antigen binding sites and is generally not a native sequence IgM or IgA antibody.

In some embodiments, the binding protein is a single chain dual variable domain immunoglobulin protein. The terms "single chain dual variable domain immunoglobulin protein" or "scDVD-Ig protein" or scFvDVD-Ig protein" refer to the antigen binding fragment of a DVD molecule that is analogous to an antibody single chain Fv fragment. scDVD-Ig proteins are described in US application 2014/0243228 and US application 2014/0221621. In an embodiment, the variable domains of a scDVD-Ig protein are antibody variable domains. In an embodiment, the variable domains are non-immunoglobulin variable domains (e.g., receptor).

In some embodiments, the binding protein is a DVD-Fab. The terms "DVD-Fab" or fDVD-Ig protein" refer to the antigen binding fragment of a DVD-Ig molecule that is analogous to an antibody Fab fragment. fDVD-Ig proteins are described in US Application 2014/0243228 and US application US 2014/0235476.

In some embodiments, the binding protein is a receptor DVD-Ig protein. The terms "receptor DVD-Ig protein" constructs, or "rDVD-Ig protein" refer to DVD-Ig^{™} constructs comprising at least one receptor-like binding domain. rDVD-Ig proteins are described in US application 2014/0219913.

The term "receptor domain" (RD), or receptor binding domain refers to the portion of a cell surface receptor, cytoplasmic receptor, nuclear receptor, or soluble receptor that functions to bind one or more receptor ligands or signaling molecules (e.g., toxins, hormones, neurotransmitters, cytokines, growth factors, or cell recognition molecules).

The terms multi-specific and multivalent IgG-like molecules or "pDVD-Ig" proteins are capable of binding two or more proteins (e.g., antigens). pDVD-Ig proteins are described in US Application 2014/0213771. In certain embodiments, pDVD-IG^{™} proteins are disclosed which are generated by specifically modifying and adapting several concepts. These concepts include but are not limited to: (1) forming Fc heterodimer using CH3 "knobs-into-holes" design, (2) reducing light chain missing pairing by using CH1/CL cross-over, and (3) pairing two separate half IgG molecules at protein production stage using "reduction then oxidation" approach.

In certain embodiments, a binding protein disclosed herein is a "half-DVD-Ig" comprised of one DVD-Ig heavy chain and one DVD-Ig light chain. The half-DVD-Ig^{™} protein preferably does not promote cross-linking observed with naturally occurring antibodies which can result in antigen clustering and undesirable activities. See U.S. Patent Publication No. 2012/0201746. In some embodiments, the binding protein is a pDVD-Ig protein. In one embodiment, a pDVD-Ig construct may be created by combining two halves of different DVD-Ig molecules, or a half DVD-Ig protein and half IgG molecule.

In some embodiments, the binding protein is an mDVD-Ig protein. As used herein "monobody DVD-Ig protein" or "mDVD-Ig protein" refers to a class of binding molecules wherein one binding arm has been rendered non-functional. mDVD-Ig proteins are described in US Application 2014/0221622.

The Fc regions of the two polypeptide chains that have a formula of VDH-(X1)n-C-(X2)n may each contain a mutation, wherein the mutations on the two Fc regions enhance heterodimerization of the two polypeptide chains. In one aspect, knobs-into-holes mutations may be introduced into these Fc regions to achieve heterodimerization of the Fc regions. See Atwell et al. (1997) J. Mol. Biol. 270:26-35.

In some embodiments, the binding protein is a cross-over DVD-Ig protein. As used herein "cross-over DVD-Ig" protein or "coDVD-Ig" protein refers to a DVD-Ig protein wherein the cross-over of variable domains is used to resolve the issue of affinity loss in the inner antigen-binding domains of some DVD-Ig molecules. coDVD-Ig proteins are described in US Publication No. 20140213772A1.

In certain embodiments, a binding protein that binds to TIGIT, PD-1, TIM-3, LAG-3, or CTLA-4 is provided as part of a bispecific antibody. The term "bispecific antibody", as used herein, refers to full-length antibodies that are generated by quadroma technology (see Milstein et al. (1983) Nature 305: 537-540), by chemical conjugation of two different monoclonal antibodies (see Staerz et al. (1985) Nature 314: 628-631), or by knob-into-hole or similar approaches which introduces mutations in the Fc region (see Holliger et al. (1993) Proc. Natl. Acad. Sci. USA 90(14): 6444-6448), resulting in multiple different immunoglobulin species of which only one is the functional bispecific antibody. By molecular function, a bispecific antibody binds one antigen (or epitope) on one of its two binding arms (one pair of HC/LC), and binds a different antigen (or epitope) on its second arm (a different pair of HC/LC). By this definition, a bispecific antibody has two distinct antigen binding arms (in both specificity and CDR sequences), and is monovalent for each antigen it binds.

The term "dual-specific antibody", as used herein, refers to full-length antibodies that can bind two different antigens (or epitopes) in each of its two binding arms (a pair of HC/LC) (see PCT Publication No. WO 02/02773). Accordingly a dual-specific binding protein has two identical antigen binding arms, with identical specificity and identical CDR sequences, and is bivalent for each antigen to which it binds.

A "functional antigen binding site" of a binding protein is one that is capable of binding a target antigen. The antigen binding affinity of the antigen binding site is not necessarily as strong as the parent antibody from which the antigen binding site is derived, but the ability to bind antigen must be measurable using any one of a variety of methods known for evaluating antibody binding to an antigen. Moreover, the antigen binding affinity of each of the antigen binding sites of a multivalent antibody herein need not be quantitatively the same.

As used herein, the terms "donor" and "donor antibody" refer to an antibody providing one or more CDRs. In an exemplary embodiment, the donor antibody is an antibody from a species different from the antibody from which the framework regions are obtained or derived. In the context of a humanized antibody, the term "donor antibody" refers to a non-human antibody providing one or more CDRs.

As used herein, the terms "acceptor" and "acceptor antibody" refer to the antibody providing or nucleic acid sequence encoding at least 80%, at least 85%, at least 90%, at least 95%, at least 98%, or 100% of the amino acid sequences of one or more of the framework regions. In some embodiments, the term "acceptor" refers to the antibody amino acid providing or nucleic acid sequence encoding the constant region(s). In yet another embodiment, the term "acceptor" refers to the antibody amino acid providing or nucleic acid sequence encoding one or more of the framework regions and the constant region(s). In a specific embodiment, the term "acceptor" refers to a human antibody amino acid or nucleic acid sequence that provides or encodes at least 80%, preferably, at least 85%, at least 90%, at least 95%, at least 98%, or 100% of the amino acid sequences of one or more of the framework regions. In accordance with this embodiment, an acceptor may contain at least 1, at least 2, at least 3, least 4, at least 5, or at least 10 amino acid residues that does (do) not occur at one or more specific positions of a human antibody. An acceptor framework region and/or acceptor constant region(s) may be, e.g., derived or obtained from a germline antibody gene, a mature antibody gene, a functional antibody (e.g., antibodies well known in the art, antibodies in development, or antibodies commercially available).

Human heavy chain and light chain acceptor sequences are known in the art. In one embodiment of the disclosure the human heavy chain and light chain acceptor sequences are selected from the sequences listed from V-base (http://vbase.mrc-cpe.cam.ac.uk/) or from IMGT^{™} the international ImMunoGeneTics Information System^{™}. (http://imgt.cines.fr/textes/IMGTrepertoire/LocusGenes/). In another embodiment of the disclosure the human heavy chain and light chain acceptor sequences are selected from the sequences described in Table 3 and Table 4 of U.S. Patent Publication No. 2011/0280800.

As used herein, the term "germline antibody gene" or "gene fragment" refers to an immunoglobulin sequence encoded by non-lymphoid cells that have not undergone the maturation process that leads to genetic rearrangement and mutation for expression of a particular immunoglobulin. (See, e.g., Shapiro et al. (2002) Crit. Rev. Immunol. 22(3): 183-200; Marchalonis et al. (2001) Adv. Exp. Med. Biol. 484:13-30). One of the advantages provided by various embodiments of the present disclosure stems from the recognition that germline antibody genes are more likely than mature antibody genes to conserve essential amino acid sequence structures characteristic of individuals in the species, hence less likely to be recognized as from a foreign source when used therapeutically in that species.

As used herein, the term "key" residues refer to certain residues within the variable domain that have more impact on the binding specificity and/or affinity of an antibody, in particular a humanized antibody. A key residue includes, but is not limited to, one or more of the following: a residue that is adjacent to a CDR, a potential glycosylation site (can be either N- or O-glycosylation site), a rare residue, a residue capable of interacting with the antigen, a residue capable of interacting with a CDR, a canonical residue, a contact residue between heavy chain variable domain and light chain variable domain, a residue within the Vernier zone, and a residue in the region that overlaps between the Chothia definition of a variable heavy chain CDR/and the Kabat definition of the first heavy chain framework.

The term "Framework regions" (hereinafter "FR") refers to the variable domain residues that are not the CDR residues. Because the exact definition of a CDR sequence can be determined by different systems, the meaning of a framework sequence is subject to correspondingly different interpretations. The six CDRs (CDR-L1, -L2, and -L3 of light chain and CDR-H1, -H2, and -H3 of heavy chain) also divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. Without specifying the particular sub-regions as FR1, FR2, FR3 or FR4, a framework region, as referred by others, represents the combined FR's within the variable domain of a single, naturally occurring immunoglobulin chain. As used herein, a FR represents one of the four sub-regions, and FRs represents two or more of the four sub-regions constituting a framework region. Without wishing to be bound by theory, each variable domain typically has four FRs identified as FR1, FR2, FR3 and FR4. If the CDRs are defined according to Kabat, the light chain FR residues are positioned at about residues 1-23 (LCFR1), 35-49 (LCFR2), 57-88 (LCFR3), and 98-107 (LCFR4) and the heavy chain FR residues are positioned about at residues 1-30 (HCFR1), 36-49 (HCFR2), 66-94 (HCFR3), and 103-113 (HCFR4) in the heavy chain residues. If the CDRs comprise amino acid residues from hypervariable loops, the light chain FR residues are positioned about at residues 1-25 (LCFR1), 33-49 (LCFR2), 53-90 (LCFR3), and 97-107 (LCFR4) in the light chain and the heavy chain FR residues are positioned about at residues 1-25 (HCFR1), 33-52 (HCFR2), 56-95 (HCFR3), and 102-113 (HCFR4) in the heavy chain residues. In some instances, when the CDR comprises amino acids from both a CDR as defined by Kabat and those of a hypervariable loop, the FR residues will be adjusted accordingly. For example, when CDRH1 includes amino acids H26-H35, the heavy chain FR1 residues are at positions 1-25 and the FR2 residues are at positions 36-49.

An "isolated antibody" is intended to refer to an antibody that is substantially free of other antibodies having different antigenic specificities (e.g., an isolated antibody that specifically binds TIGIT, PD-1, TIM-3, LAG-3, or CTLA-4 is substantially free of antibodies that specifically bind antigens other than TIGIT, PD-1, TIM-3, LAG-3, or CTLA-4). An isolated antibody that specifically binds TIGIT, PD-1, TIM-3, LAG-3, or CTLA-4 may, however, have cross-reactivity to other antigens, such as TIGIT, PD-1, TIM-3, LAG-3, or CTLA-4 molecules from other species. Moreover, an isolated antibody may be substantially free of other cellular material and/or chemicals.

The term "diabodies" refers to small antibody fragments with two antigen-binding sites, which fragments comprise a heavy chain variable domain (V_{H}) connected to a light chain variable domain (V_{L}) in the same polypeptide chain (V_{H} and V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "linear antibodies" refers to the antibodies described in Zapata et al., Protein Eng., 8(10):1057-1062 (1995). Briefly, these antibodies comprise a pair of tandem Fd segments (V_{H} - C_{H}1-V_{H}-C_{H}1) which, together with complementary light chain polypeptides, form a pair of antigen binding regions. Linear antibodies can be bispecific or monospecific.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

The term "humanized antibody" refers to antibodies that comprise heavy and light chain variable domain sequences from a non-human species (e.g., a mouse) but in which at least a portion of the VH and/or VL sequence has been altered to be more "human-like", i.e., more similar to human germline variable sequences. Accordingly, "humanized" antibodies are a form of a chimeric antibody, that are engineered or designed to comprise minimal sequence derived from non-human immunoglobulin. For the most part, humanized antibodies are human immunoglobulins (recipient or acceptor antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies can comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). As used herein, a "composite human antibody" or "deimmunized antibody" are specific types of engineered or humanized antibodies designed to reduce or eliminate T cell epitopes from the variable domains.

One type of humanized antibody is a CDR-grafted antibody, in which human CDR sequences are introduced into non-human VH and VL sequences to replace the corresponding nonhuman CDR sequences. Also "humanized antibody" is an antibody or a variant, derivative, analog or fragment thereof which immunospecifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 80%, at least 85%, at least 90%, at least 95%, at least 98% or at least 99% identical to the amino acid sequence of a non-human antibody CDR. A humanized antibody comprises substantially all of at least one, and typically two, variable domains (Fab, Fab', F(ab').sub.2, FabC, Fv) in which all or substantially all of the CDR regions correspond to those of a non-human immunoglobulin (i.e., donor antibody) and all or substantially all of the framework regions are those of a human immunoglobulin consensus sequence. In an embodiment, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain. A humanized antibody may be selected from any class of immunoglobulins, including IgM, IgG, IgD, IgA and IgE, and any isotype including without limitation IgG1, IgG2, IgG3, and IgG4. The humanized antibody may comprise sequences from more than one class or isotype, and particular constant domains may be selected to optimize desired effector functions using techniques well known in the art.

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to either the donor antibody or the consensus framework. In an exemplary embodiment, such mutations, however, will not be extensive. Usually, at least 80%, preferably at least 85%, more preferably at least 90%, and most preferably at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (see, e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

With respect to constructing DVD-Ig or other binding protein molecules, a "linker" is used to denote a single amino acid or a polypeptide ("linker polypeptide") comprising two or more amino acid residues joined by peptide bonds and used to link one or more antigen binding portions. Such linker polypeptides are well known in the art (see, e.g., Holliger et al. (1993) Proc. Natl. Acad. Sci. USA 90: 6444-6448; Poljak (1994) Structure 2: 1121-1123).

As used herein, "Vernier" zone refers to a subset of framework residues that may adjust CDR structure and fine-tune the fit to antigen as described by Foote et al. (1992) J. Mol. Biol., 224: 487-499. Vernier zone residues form a layer underlying the CDRs and may impact on the structure of CDRs and the affinity of the antibody.

A "human antibody," "non-engineered human antibody," or "fully human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. Proc. Natl. Acad. Sci. 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous mouse immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody can be prepared via immortalization of human B lymphocytes producing an antibody directed against a target antigen (such B lymphocytes can be recovered from an individual or can have been immunized *in vitro*)*.* See, *e.g.,* Cole et al., Monoclonal Antibodies and Cancer Therapy, Alan R. Liss, p. 77 (1985); Boerner et al., J. Immunol., 147 (1):86-95 (1991); and U.S. Pat. No. 5,750,373.

An "affinity matured" antibody is one with one or more alterations in one or more CDRs thereof which result an improvement in the affinity of the antibody for antigen, compared to a parent antibody which does not possess those alteration(s). Exemplary affinity matured antibodies will have nanomolar or even picomolar affinities for the target antigen. A variety of proceedures for producing affinity matured antibodies are known in the art. For example, Marks et al. Bio/Technology 10:779-783 (1992) describes affinity maturation by V_{H} and V_{L} domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155: 1994-2004 (1995); Jackson et al., J. Immunol. 154(7):3310-9 (1995); and Hawkins et al., J. Mol. Biol. 226:889-896 (1992). Selective mutation at selective mutagenesis positions and at contact or hypermutation positions with an activity enhancing amino acid residue is described in U.S. Pat. No. 6,914,128.

A "functional antigen binding site" or "functional binding domain" of an antibody is one which is capable of binding a target antigen. The antigen binding affinity of the antigen binding site is not necessarily as strong as the parent antibody from which the antigen binding site is derived, but the ability to bind antigen must be measurable using any one of a variety of methods known for evaluating antibody binding to an antigen. Moreover, the antigen binding affinity of each of the antigen binding sites of a multivalent antibody herein need not be quantitatively the same. For multimeric antibodies, the number of functional antigen binding sites can be evaluated using ultracentrifugation analysis as described in Example 2 of U.S. Patent Application Publication No. 20050186208. According to this method of analysis, different ratios of target antigen to multimeric antibody are combined and the average molecular weight of the complexes is calculated assuming differing numbers of functional binding sites. These theoretical values are compared to the actual experimental values obtained in order to evaluate the number of functional binding sites.

As used herein, a "blocking" or "neutralizing" binding protein, antibody, antibody fragment, antigen-binding fragment or an antibody "antagonist" is one which inhibits or reduces the biological activity of the antigen it specifically binds to the antigen. In certain embodiments, blocking or neutralizing antibodies or antagonist antibodies completely inhibit the biological activity of the antigen. A neutralizing binding protein, antibody, antigen-binding fragment thereof as described herein can bind to TIGIT, PD-1, TIM-3, LAG-3, or CTLA-4 resulting in the inhibition of a biological activity of TIGIT, PD-1, TIM-3, LAG-3, or CTLA-4.

As used herein, "small molecule inhibitors" include, but are not limited to, small peptides or peptide-like molecules, soluble peptides, and synthetic non-peptidyl organic or inorganic compounds. A small molecule inhibitor or antagonist can have a molecular weight of any of about 100 to about 20,000 daltons (Da), about 500 to about 15,000 Da, about 1000 to about 10,000 Da.

The term "therapeutically effective amount" therefore refers to an amount of the inhibitors or potentiators described herein, using the methods as disclosed herein, that is sufficient to provide a particular effect when administered to a typical subject. An effective amount as used herein would also include an amount sufficient to delay the development of a symptom of the disease, alter the course of a symptom disease (for example but not limited to, slow the progression of a symptom of the disease), or reverse a symptom of the disease. Thus, it is not possible to specify the exact "effective amount". However, for any given case, an appropriate "effective amount" can be determined by one of ordinary skill in the art using only routine experimentation.

A "cancer" or "tumor" as used herein refers to an uncontrolled growth of cells which interferes with the normal functioning of the bodily organs and systems. A subject that has a cancer or a tumor is a subject having objectively measurable cancer cells present in the subject's body. Included in this definition are benign tumors and malignant cancers, as well as dormant tumors or micrometastases. Cancers which migrate from their original location and seed vital organs can eventually lead to the death of the subject through the functional deterioration of the affected organs. Hemopoietic cancers, such as leukemia, are able to out-compete the normal hemopoietic compartments in a subject, thereby leading to hemopoietic failure (in the form of anemia, thrombocytopenia and neutropenia) ultimately causing death.

The term "anti-cancer therapy" refers to a therapy useful in treating cancer. Examples of anti-cancer therapeutic agents include, but are not limited to, e.g., surgery, chemotherapeutic agents, growth inhibitory agents, cytotoxic agents, agents used in radiation therapy, anti-angiogenesis agents, apoptotic agents, anti-tubulin agents, and other agents to treat cancer, such as anti-HER2 antibodies (e.g., HERCEPTIN^{®}), anti-CD20 antibodies, an epidermal growth factor receptor (EGFR) antagonist *(e.g.,* a tyrosine kinase inhibitor), HER1/EGFR inhibitor *(e.g.,* erlotinib (TARCEVA^{®})), platelet derived growth factor inhibitors *(e.g.,* GLEEVEC^{™} (Imatinib Mesylate)), a COX2 inhibitor *(e.g.,* celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also specifically contemplated for the methods described herein.

As used herein, the terms "tumor antigen" and "cancer antigen" are used interchangeably to refer to antigens which are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent *(i.e.,* not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), and fusion proteins resulting from internal deletions or chromosomal translocations. Still other cancer antigens can be encoded by viral genes such as those carried on RNA and DNA tumor viruses. Many tumor antigens have been defined in terms of multiple solid tumors: MAGE 1, 2, & 3, defined by immunity; MART-1/Melan-A, gp100, carcinoembryonic antigen (CEA), HER2, mucins *(i.e.,* MUC-1), prostate-specific antigen (PSA), and prostatic acid phosphatase (PAP). In addition, viral proteins such as hepatitis B (HBV), Epstein-Barr (EBV), and human papilloma (HPV) have been shown to be important in the development of hepatocellular carcinoma, lymphoma, and cervical cancer, respectively. However, due to the immunosuppression of patients diagnosed with cancer, the immune systems of these patients often fail to respond to the tumor antigens.

As used herein, an "autoimmune disease" refers to a class of diseases in which a subject's own antibodies react with host tissue or in which immune effector T cells are autoreactive to endogenous self-peptides and cause inflammation and/or destruction of tissue. Thus an immune response is mounted against a subject's own antigens, referred to as self-antigens. A "self-antigen" as used herein refers to an antigen of a normal host tissue. Normal host tissue does not include cancer cells.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

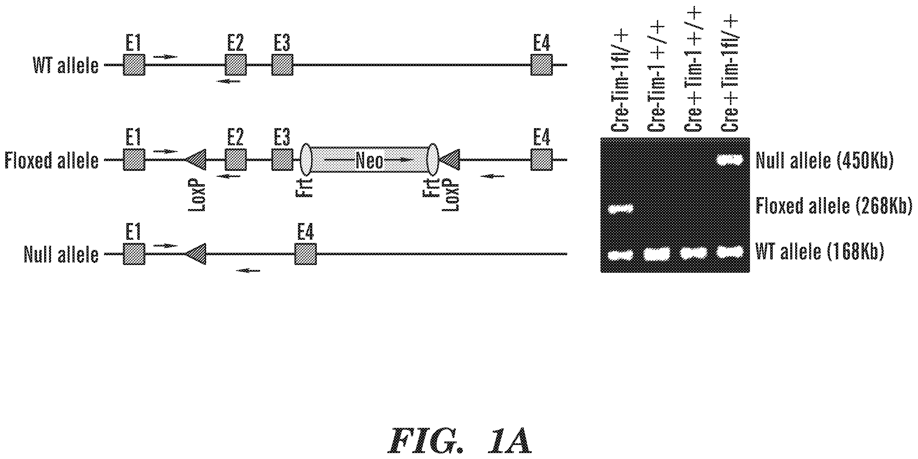

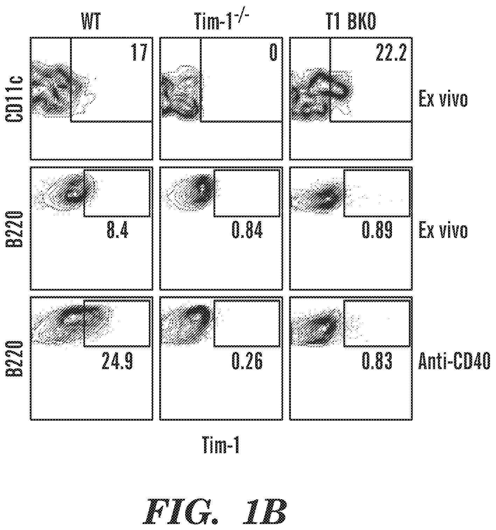

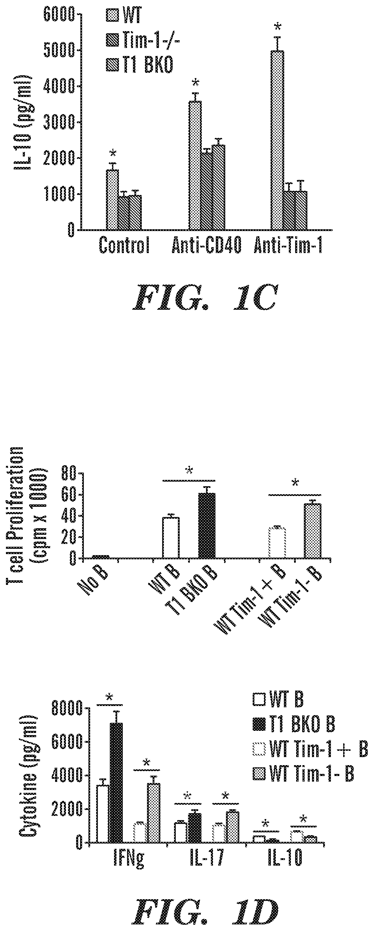

**FIGS. 1A-1D** demonstrate generation of Tim-1BKO (Tim-1 B cell-specific knockout mice) and that Tim-1 deficiency impaired Breg IL-10 production and suppressive function. **FIG. 1A****.** Strategy for generation of Tim-1 conditional knockout mice. **FIG. 1B****.** Tim-1 expression in WT, Tim-1-/-, and T1BKO mice. **FIG. 1C****.** Normal B cell populations in T1BKO mice but B cells from T1BKO mice showed impaired IL-10 production. **FIG. 1D****.B** cells from T1BKO mice promoted T cell proliferation and inflammatory cytokine production.

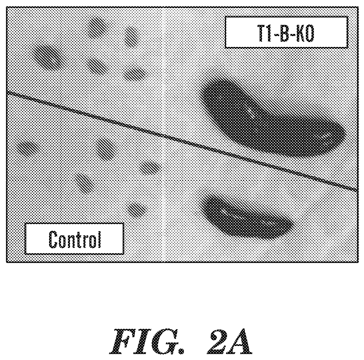

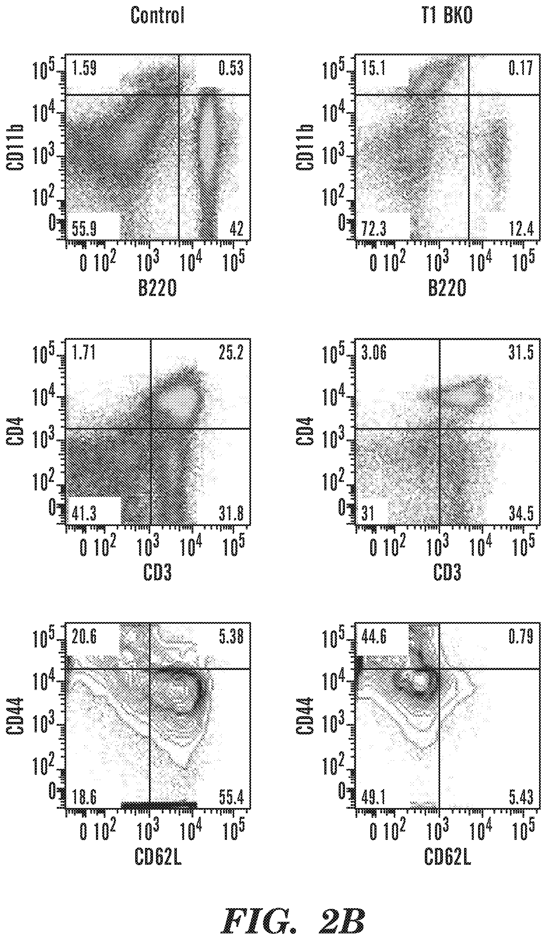

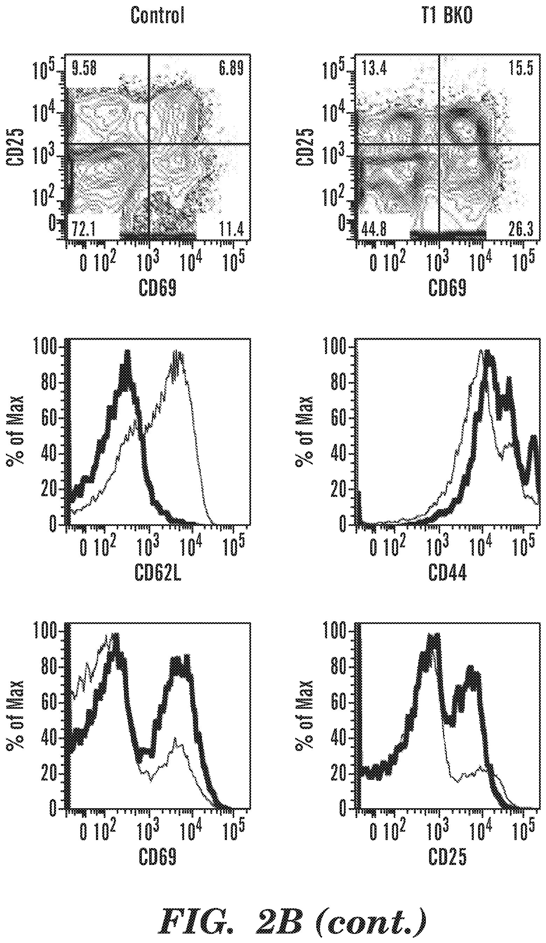

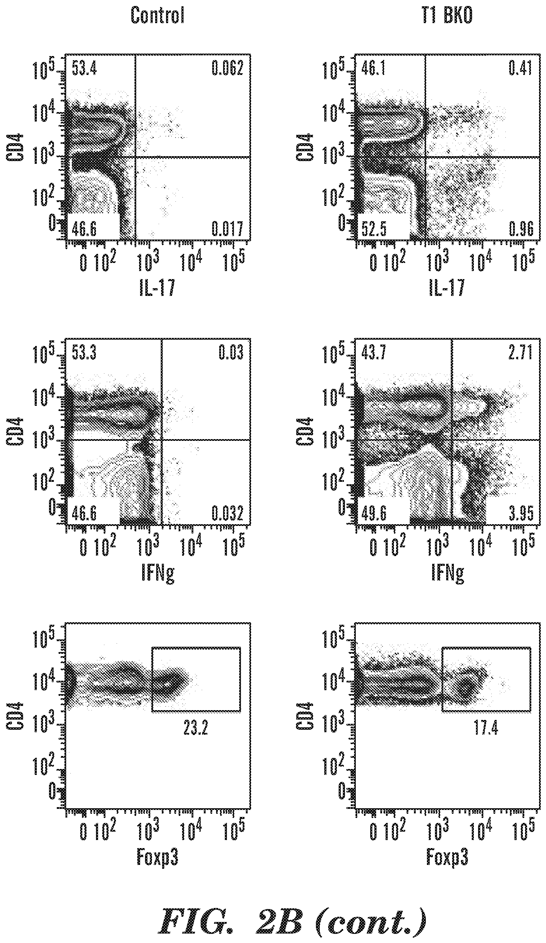

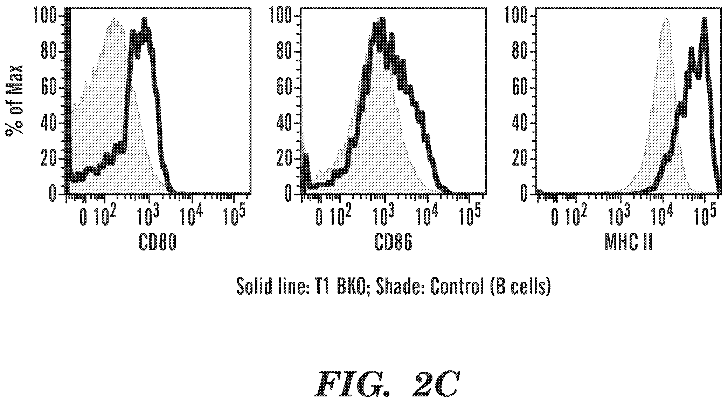

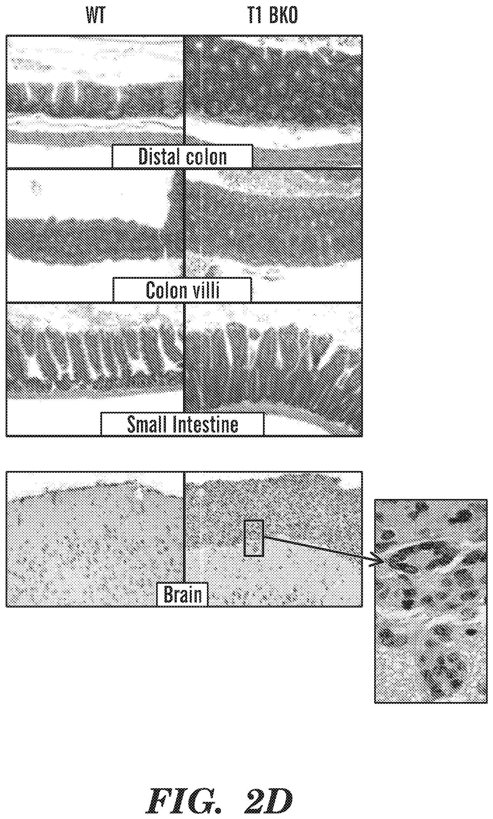

**FIGS. 2A-2D** demonstrate Tim-1BKO mice develop spontaneous inflammation in multiple organs and tissues with age. **FIG. 2A****.** Enlarged spleen with increased T cells and CD11b+ cells from T1BKO mice at 10-12 months of age. **FIG. 2B****.** T cell phenotypes in the old T1BKO mice. **FIG. 2C****.** B cell activation in the old T1BKO mice. **FIG. 2D****.** Histology of multiple organs and tissues in old T1BKO mice.

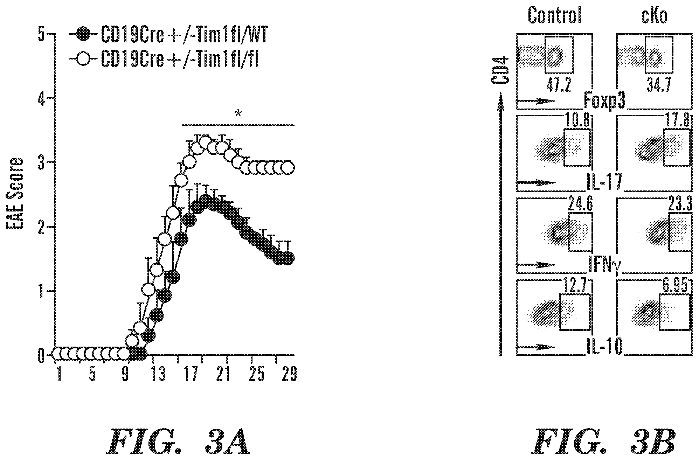

**FIGS. 3A-3B** demonstrate young Tim-1BKO mice develop more severe MOG35-55-induced EAE (experimental autoimmune encephamyelitis). **FIG. 3A****.** Mice at 6-8 weeks of age were immunized with MOG35-55/CFA, and scored daily for clinical signs of EAE (n=8 per group). *, P<0.05; **FIG. 3B****.** T cell responses in the CNS (central nervous system).

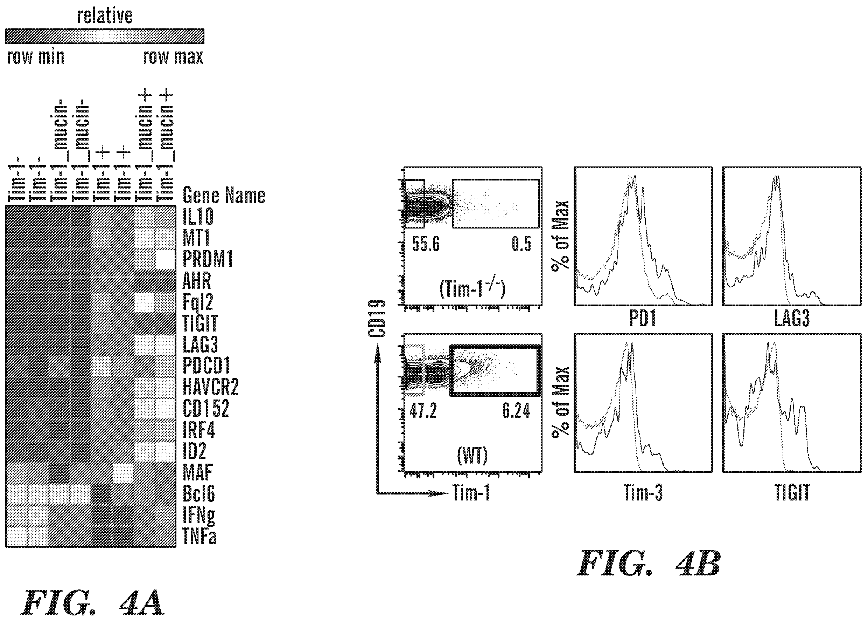

**FIGS. 4A-4B** demonstrate that Tim-1+ B cells highly express IL-10 as well as a unique set of coinhibitory molecules and transcription factors. Nanostring analysis of splenic Tim-1+ and Tim-1- B cells from 8 week-old naive WT and Tim-1Δmucin mice and phenotypes of Tim-1+ vs. Tim-1- B cells. **FIG. 4A****.** Heatmap showing selected differential expressed genes. **FIG. 4B****.** Representative FACS plots showing expression of selected coinhibitory molecules by Tim-1+ vs.Tim-1- B cells from 8 wk-old naive WT mice. Red line: Tim-1-/- and Blue line: Tim-1+ B cells.

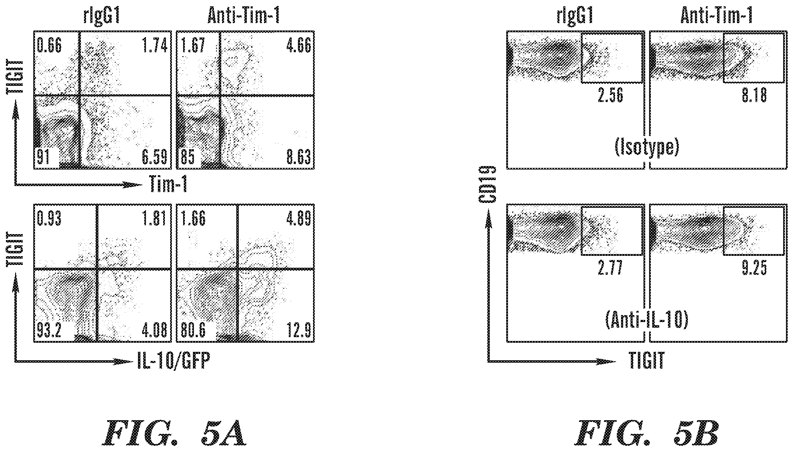

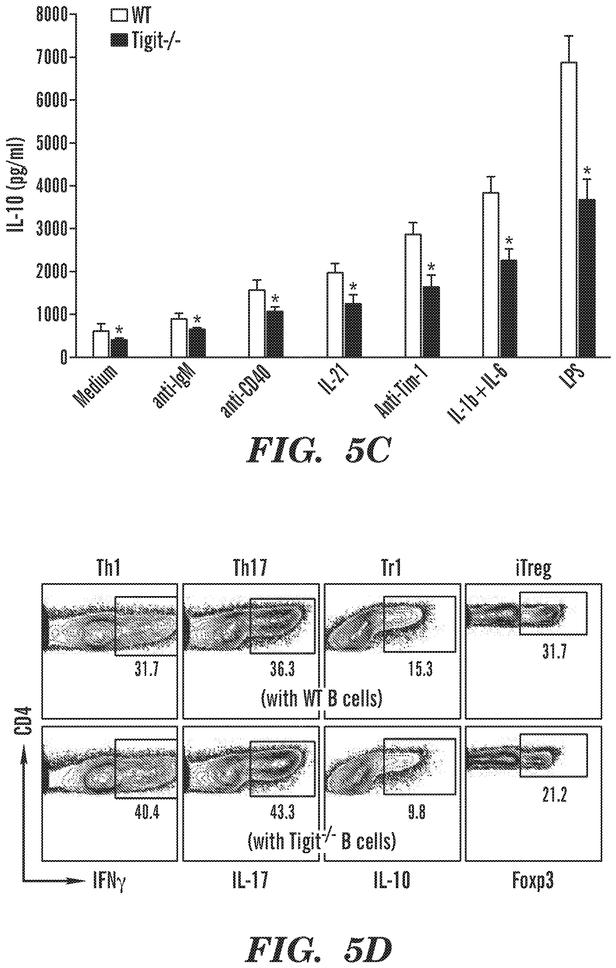

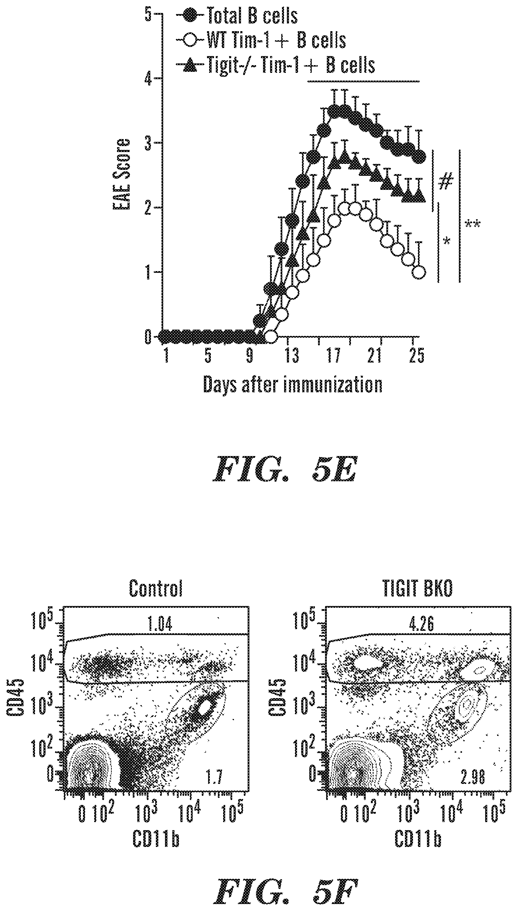

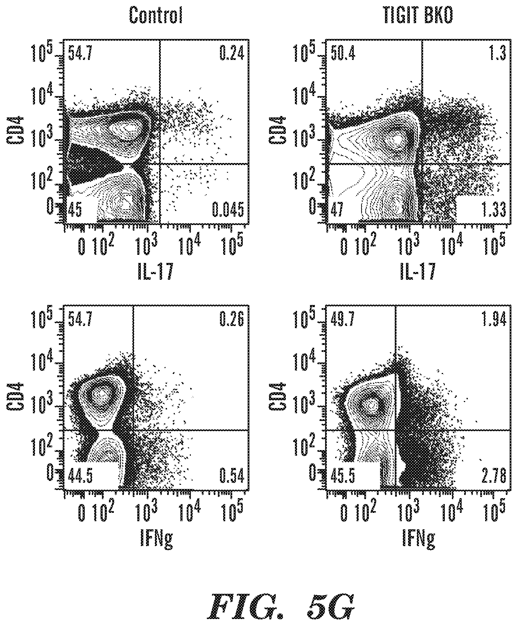

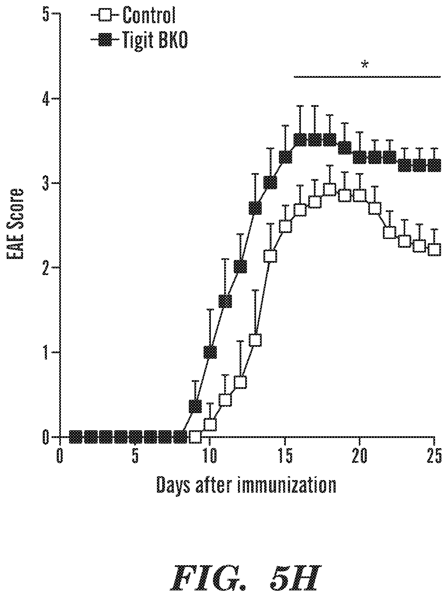

**FIGS. 5A-5H** demonstrate TIGIT expression and role in Bregs. **FIG. 5A****.** Tim-1, TIGIT, and IL-10-GFP expression in splenic CD19+ B cells from Tiger mice determined by flow cytometry after cells were treated with anti-Tim-1 or control rIgG1 for 3 days. **FIG. 5B****.** WT B cells were treated with anti-Tim-1 or control rIgG1 with or without anti-IL-10 blocking antibodies for 3 days, and then measured for TIGIT expression. **FIG. 5C****.** B cells from WT and Tigit-/- mice were treated with indicated stimuli for 3 days, and IL-10 production in the cultures was measured by ELISA. * P<0.01. **FIG. 5D****.** WT and Tigit-/-splenic CD19+ B cells were co-cultured with naive CD4+ T cells in the presence of anti-CD3 for 4 days, and cytokine production was determined by flow cytometry. **FIG. 5E****.** Total WT B cells, WT Tim-1+ Bregs, or Tigit-/- Tim-1+ Bregs (2 x 10⁶ /mouse) were transferred into WT mice; the recipients were then immunized with MOG35-55/CFA to induce EAE. Mice were scored daily for clinical signs of EAE (n =8 per group). *, ** P<0.01; #P<0.05. Representative FACS plots show increased infiltration of CD45+ leukocytes in the brain **(****FIG. 5F****)** and increased IFN-γ and IL-17 production in T cells (gated on CD3+ cells) **(****FIG. 5G****)** from a TIGIT BKO mouse with spontaneous EAE-like symptoms; **FIG. 5H****.** Control and TIGIT BKO mice (8 wk-old; n=10/group) were immunized with MOG₃₅₋₅₅/CFA and monitored daily for clinical signs of EAE. * P<0.05.

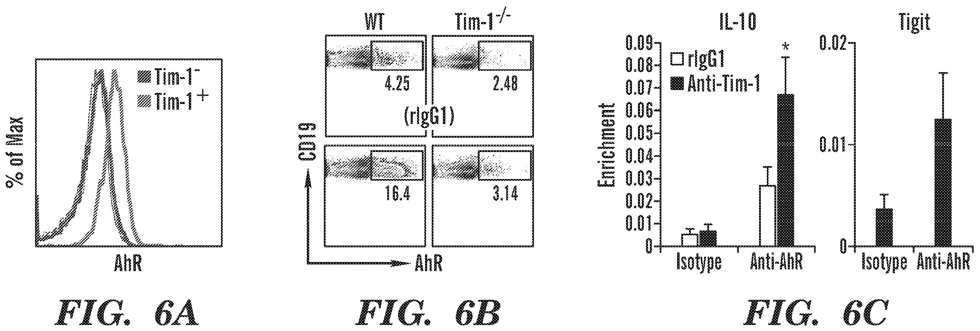

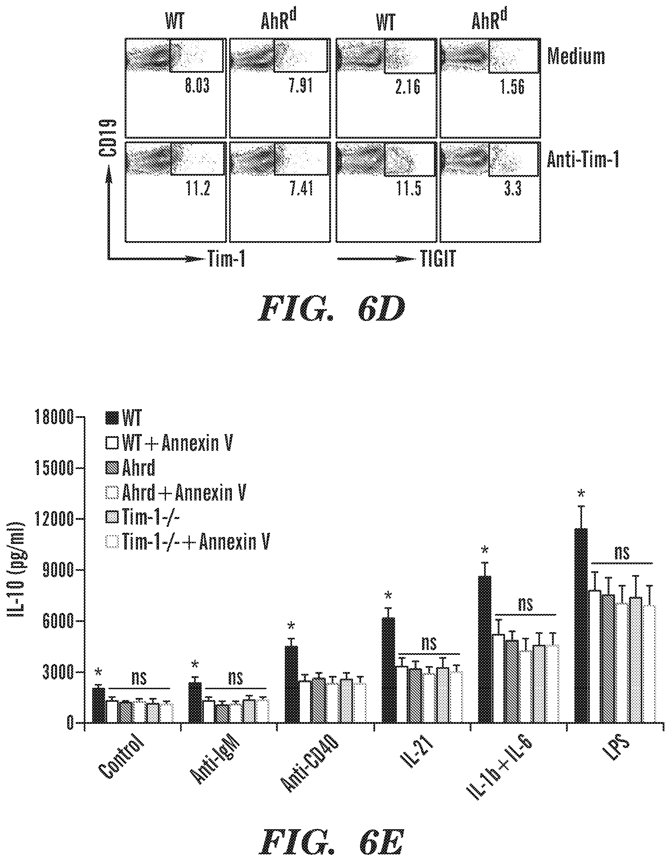

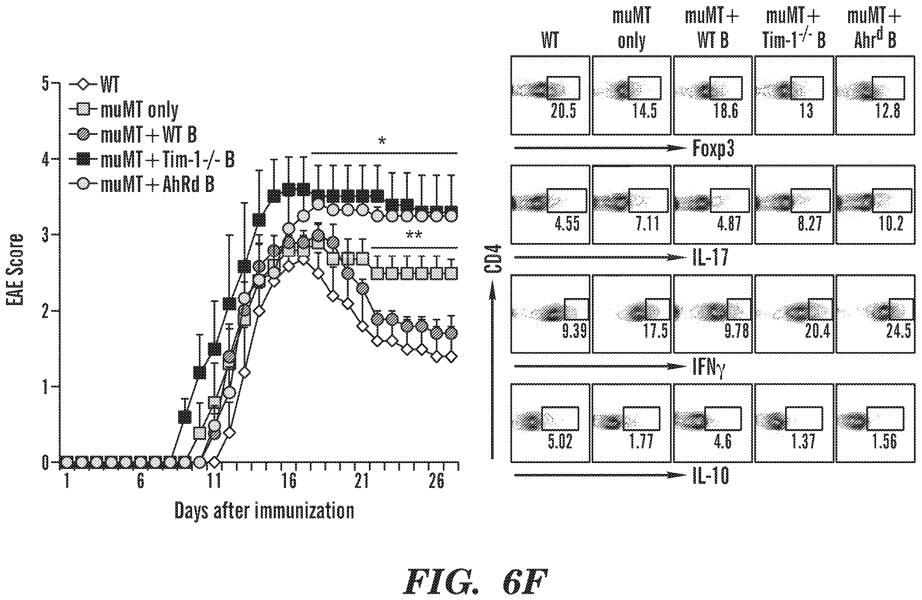

**FIGS. 6A-6F** demonstrate roles of AhR in Bregs. **FIG. 6A****.** AhR expression as determined by flow cytometry in Tim-1- and Tim-1+ WT B cells; Thin line, isotype stain; thick line, anti-AhR stain. **FIG. 6B****.** WT and Tim-1-/- B cells were treated with anti-Tim-1 or control rIgG1 for 3 days, and then measured for AhR expression. **FIG. 6C****.** AhR ChIP-PCR in the XRE in the IL10 and Tigit promoters in WT B cells treated with anti-Tim-1 or rIgG1; * P<0.01. **FIG. 6D****.** TIGIT and IL-10 expression was determined by flow cytometry in WT and Tim-1-/- B cells 3 days after anti-Tim-1 or control rIgG1 for 3 days. **FIG. 6E****.** WT, AhRd, and Tim-1-/- B cells were treated with indicated stimuli with or without Annexin V for 3 days, and IL-10 production in the cultures was measured by ELISA. * P<0.01. **FIG. 6F****.** WT, AhRd, and Tim-1-/- B cells were transferred into B cell deficient mice and then induced EAE with MOG35-55/CFA. Shown are the clinical EAE score (left panel) and CNS-infiltrating T cell phenotypes (right panel).

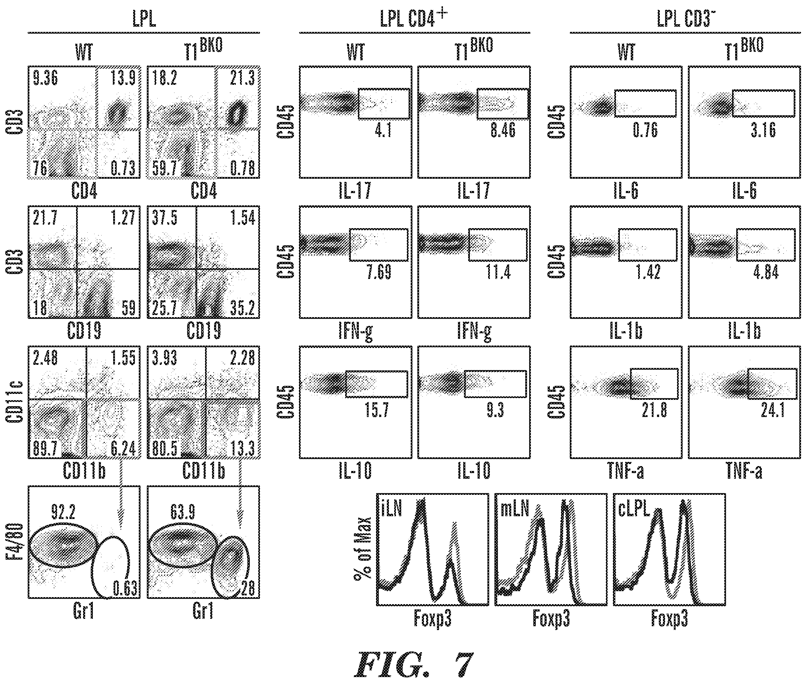

**FIG. 7** shows T cells from Tim-1BKO produced more IFN-γ and IL-17 but less IL-10 than wild-type T cells.

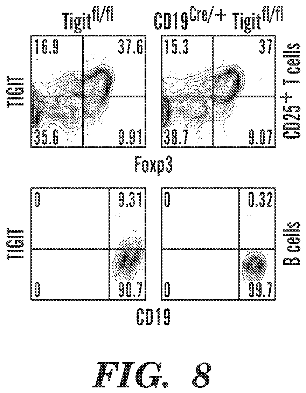

**FIG. 8** shows confirmation of efficient TIGIT deletion in B cells by flow cytometry mice.

### DETAILED DESCRIPTION

The discoveries described herein demonstrate that, in addition to being critical for restraining inflammation in induced-disease settings, regulatory B cells or Bregs are also critical and essential in maintaining self tolerance at the steady state in unmanipulated mice, and that Bregs differentially express specific coinhibitory molecules, including TIGIT, PD-1, LAG-3, TIM-3 and CTLA-4. As shown herein, TIGIT, a coinhibitory molecule differentially expressed in Bregs and regulated by Tim-1 signaling, is not only critical for Bregs in suppressing inflammation, by regulating the balance of pathogenic Th1/Th17 cells and Foxp3+ /IL-10+ regulatory T cells, it is also required for Breg-mediated tolerance maintenance, preferentially in brain. Accordingly, provided herein are methods for modulating Breg-mediated immune suppression and compositions thereof.

### Regulators of B Cell-Mediated Immunosuppression and Methods Thereof

The oxmpositions and methods described herein target one or nore regulators of B cell-mediated immunosuppression selected from TIGIT, PD-1, LAG-3, TIM-3 and CTLA-4.

The term "TIGIT" or "T-Cell Immunoreceptor With Ig And ITIM Domains," as used herein, refers to the 244 amino acid polypeptide having the amino acid sequence of:
MRWCLLLIWAQGLRQAPLASGMMTGTIETTGNISAEKGGSIILQCHLSSTTAQVTQVNWEQQ DQLLAICNADLGWHISPSFKDRVAPGPGLGLTLQSLTVNDTGEYFCIYHTYPDGTYTGRIFLE VLESSVAEHGARFQIPLLGAMAATLVVICTAVIVVVALTRKKKALRIHSVEGDLRRKSAGQE EWSPSAPSPPGSCVQAEAAPAGLCGEQRGEDCAELHDYFNVLSYRSLGNCSFFTETG (SEQ ID NO: 1), as described by, e.g., NP_776160, together with any naturally occurring allelic, splice variants, and processed forms thereof. The 21 amino acid signal peptide sequence of TIGIT is underlined for reference. Typically, TIGIT refers to human TIGIT. The sequence of TIGIT, without the 21 amino acid signal peptide sequence, is provided herein as:
MMTGTIETTGNISAEKGGSIILQCHLSSTTAQVTQVNWEQQDQLLAICNADLGWHISPSFKDR VAPGPGLGLTLQSLTVNDTGEYFCIYHTYPDGTYTGRIFLEVLESSVAEHGARFQIPLLGAMA ATLVVICTAVIVVVALTRKKKALRIHSVEGDLRRKSAGQEEWSPSAPSPPGSCVQAEAAPAG LCGEQRGEDCAELHDYFNVLSYRSLGNCSFFTETG **(SEQ ID NO: 2).** The term "TIGIT" can also, in some embodiments, be used to refer to truncated forms or fragments of the TIGIT polypeptide that retain a TIGIT biological function or activity of interest as described herein. Reference to any such forms of TIGIT can be identified in the application, e.g., by "amino acids 25-50 of SEQ ID NO: 2." Specific residues of TIGIT can be referred to as, for example, "TIGIT(25)," with reference to SEQ ID NO: 2 implicit.

PD-1 (or CD279) is a 288 amino acid type I transmembrane protein composed of one immunoglobulin (Ig) superfamily domain, a 20 amino acid stalk, a transmembrane domain, and an intracellular domain of approximately 95 residues containing an immunoreceptor tyrosine-based inhibitory motif (ITIM), as well as an immunoreceptor tyrosine-based switch motif (ITSM).

Splice variants of PD-1 have been cloned from activated human T cells. These transcripts lack exon 2, exon 3, exons 2 and 3, or exons 2 through 4. All these variants, except for the splice variant lacking exon 3 only (PD-1Δex3), are expressed at similar levels as full-length PD-1 in resting peripheral blood mononuclear cells (PBMCs). All variants are significantly induced upon activation of human T cells with anti-CD3 and anti-CD28 (Keir ME et al., 2008. Annu Rev Immunol. 26:677-704).
Accordingly, the term " PD-1" as used herein, refers to the 288 amino acid polypeptide having the amino acid sequence of:
MQIPQAPWPVVWAVLQLGWRPGWFLDSPDRPWNPPTFSPALLVVTEGDNATFTCSFSNTSES FVLNWYRMSPSNQTDKLAAFPEDRSQPGQDCRFRVTQLPNGRDFHMSVVRARRNDSGTYLC GAISLAPKAQIKESLRAELRVTERRAEVPTAHPSPSPRPAGQFQTLVVGVVGGLLGSLVLLVW VLAVICSRAARGTIGARRTGQPLKEDPSAVPVFSVDYGELDFQWREKTPEPPVPCVPEQTEYA TIVFPSGMGTSSPARRGSADGPRSAQPLRPEDGHCSWPL **(SEQ ID NO: 3),** as described by, *e.g.,* NP_005009, together with any naturally occurring allelic, splice variants, and processed forms thereof. Typically, PD-1 refers to human PD-1. The sequence of PD-1, without the 20 amino acid signal peptide sequence, is provided herein as:
PGWFLDSPDRPWNPPTFSPALLVVTEGDNATFTCSFSNTSESFVLNWYRMSPSNQTDKLAAF PEDRSQPGQDCRFRVTQLPNGRDFHMSVVRARRNDSGTYLCGAISLAPKAQIKESLRAELRV TERRAEVPTAHPSPSPRPAGQFQTLVVGVVGGLLGSLVLLVWVLAVICSRAARGTIGARRTG QPLKEDPSAVPVFSVDYGELDFQWREKTPEPPVPCVPEQTEYATIVFPSGMGTSSPARRGSAD GPRSAQPLRPEDGHCSWPL **(SEQ ID NO: 4).** The term "PD-1" is also used to refer to truncated forms or fragments of the PD-1 polypeptide. Reference to any such forms of PD-1 can be identified in the application, e.g., by "PD-1 (42-136)," or the PD-1 IgV domain as PD-1(42-136). Specific residues of PD-1 can be referred to as, for example, "PD-1(68)," with reference to SEQ ID NO: 4 implicit.

PD-1 has two known ligands, PD-L1 and PD-L2, which are also members of the B7 family. The binding interface of PD-1 to PD-L1 is via its IgV-like domain *(i.e.,* PD-1(42-136)). Residues important for binding of PD-1 to its ligands include residues 64, 66, 68, 73, 74, 75, 76, 78, 90, 122, 124, 126, 128, 130, 131, 132, 134, and 136. Residues of PD-L1 important for binding to PD-1 include PD-L1(67), PD-L1(121), PD-L1(122), PD-L1(123), PD-L1(123), PD-L1(124), and PD-L1(126).

TIM-3 is a Type I cell-surface glycoprotein that comprises an N-terminal immunoglobulin (Ig)-like domain, a mucin domain with O-linked glycosylations and with N-linked glycosylations close to the membrane, a single transmembrane domain, and a cytoplasmic region with tyrosine phosphorylation motif(s). TIM-3 is a member of the T cell/transmembrane, immunoglobulin, and mucin (TIM) gene family.
Accordingly, the term " TIM-3" as used herein, refers to the 301 amino acid polypeptide having the amino acid sequence of: 
MFSHLPFDCVLLLLLLLLTRSSEVEYRAEVGQNAYLPCFYTPAAPGNLVPVCWGKGACPVFE
CGNVVLRTDERDVNYWTSRYWLNGDFRKGDVSLTIENVTLADSGIYCCRIQIPGIMNDEKFN
LKLVIKPAKVTPAPTLQRDFTAAFPRMLTTRGHGPPAETQTLGSLPDINLTQISTLANELRDSR
LANDLRDSGATIRIGIYIGAGICAGLALALIFGALIFKWYSHSKEKIQNLSLISLANLPPSGLAN
AVAEGIRSEENIYTIEENVYEVEEPNEYYCYVSSRQQPSQPLGCRFAMP (SEQ ID NO: 5),
 as described by, e.g., AAL65157, together with any naturally occurring allelic, splice variants, and processed forms thereof. Typically, TIM-3 refers to human TIM-3. The term "TIM-3" is also used to refer to truncated forms or fragments of the TIM-3 polypeptide. Reference to any such forms or fragments of TIM-3 can be identified in the application, e.g., by "TIM-3 (24-131)." Specific residues of TIM-3 can be referred to as, for example, "TIM-3(62)," with reference to SEQ ID NO: 5 implicit.

The term "LAG-3" or "lymphocyte activation gene 3," as used herein, refers to the 525 amino acid polypeptide having the amino acid sequence of:
MWEAQFLGLLFLQPLWVAPVKPLQPGAEVPVVWAQEGAPAQLPCSPTIPLQDLSLLRRAGV TWQHQPDSGPPAAAPGHPLAPGPHPAAPSSWGPRPRRYTVLSVGPGGLRSGRLPLQPRVQLD ERGRQRGDFSLWLRPARRADAGEYRAAVHLRDRALSCRLRLRLGQASMTASPPGSLRASDW VILNCSFSRPDRPASVHWFRNRGQGRVPVRESPHHHLAESFLFLPQVSPMDSGPWGCILTYRD GFNVSIMYNLTVLGLEPPTPLTVYAGAGSRVGLPCRLPAGVGTRSFLTAKWTPPGGGPDLLV TGDNGDFTLRLEDVSQAQAGTYTCHIHLQEQQLNATVTLAIITVTPKSFGSPGSLGKLLCEVT PVSGQERFVWSSLDTPSQRSFSGPWLEAQEAQLLSQPWQCQLYQGERLLGAAVYFTELSSPG AQRSGRAPGALPAGHLLLFLILGVLSLLLLVTGAFGFHLWRRQWRPRRFSALEQGIHPPQAQS KIEELEQEPEPEPEPEPEPEPEPEPEQL (SEQ ID NO: 6), as described by, e.g., NP_002277.4, together with any naturally occurring allelic, splice variants, and processed forms thereof. The 22 amino acid signal peptide sequence of LAG-3 is underlined for reference. Typically, LAG-3 refers to human LAG-3. The sequence of LAG-3, without the 22 amino acid signal peptide sequence, is provided herein as:
LQPGAEVPVVWAQEGAPAQLPCSPTIPLQDLSLLRRAGVTWQHQPDSGPPAAAPGHPLAPGP HPAAPSSWGPRPRRYTVLSVGPGGLRSGRLPLQPRVQLDERGRQRGDFSLWLRPARRADAG EYRAAVHLRDRALSCRLRLRLGQASMTASPPGSLRASDWVILNCSFSRPDRPASVHWFRNRG QGRVPVRESPHHHLAESFLFLPQVSPMDSGPWGCILTYRDGFNVSIMYNLTVLGLEPPTPLTV YAGAGSRVGLPCRLPAGVGTRSFLTAKWTPPGGGPDLLVTGDNGDFTLRLEDVSQAQAGTY TCHIHLQEQQLNATVTLAIITVTPKSFGSPGSLGKLLCEVTPVSGQERFVWSSLDTPSQRSFSG PWLEAQEAQLLSQPWQCQLYQGERLLGAAVYFTELSSPGAQRSGRAPGALPAGHLLLFLILG VLSLLLLVTGAFGFHLWRRQWRPRRFSALEQGIHPPQAQSKIEELEQEPEPEPEPEPEPEPEPEP EQL **(SEQ ID NO: 7).** The term "LAG-3" can also, in some embodiments, be used to refer to truncated forms or fragments of the LAG-3 polypeptide that retain a LAG-3 biological function or activity of interest as described herein. Reference to any such forms of LAG-3 can be identified in the application, e.g., by "amino acids 25-50 of SEQ ID NO: 7." Specific residues of LAG-3 can be referred to as, for example, "LAG-3(25)," with reference to SEQ ID NO: 7 implicit.

The term "CTLA-4" or "cytotoxic T-lymphocyte protein 4," as used herein, refers to the 223 amino acid polypeptide having the amino acid sequence of:
MACLGFQRHKAQLNLATRTWPCTLLFFLLFIPVFCKAMHVAQPAVVLASSRGIASFVCEYAS PGKATEVRVTVLRQADSQVTEVCAATYMMGNELTFLDDSICTGTSSGNQVNLTIQGLRAMD TGLYICKVELMYPPPYYLGIGNGTQIYVIDPEPCPDSDFLLWILAAVSSGLFFYSFLLTAVSLSK MLKKRSPLTTGVYVKMPPTEPECEKQFQPYFIPIN **(SEQ ID NO: 8),** as described by, e.g., NP_005205.2, together with any naturally occurring allelic, splice variants, and processed forms thereof. The 35 amino acid signal peptide sequence of CTLA-4is underlined for reference. Typically, CTLA-4 refers to human CTLA-4. The sequence of CTLA-4, without the 35 amino acid signal peptide sequence, is provided herein as: 
KAMHVAQPAVVLASSRGIASFVCEYASPGKATEVRVTVLRQADSQVTEVCAATYMMGNEL
TFLDDSICTGTSSGNQVNLTIQGLRAMDTGLYICKVELMYPPPYYLGIGNGTQIYVIDPEPCPD
SDFLLWILAAVSSGLFFYSFLLTAVSLSKMLKKRSPLTTGVYVKMPPTEPECEKQFQPYFIPIN
(SEQ ID NO: 9).
 The term " CTLA-4" can also, in some embodiments, be used to refer to truncated forms or fragments of the CTLA-4 polypeptide that retain a CTLA-4 biological function or activity of interest as described herein. Reference to any such forms of CTLA-4 can be identified in the application, e.g., by "amino acids 25-50 of SEQ ID NO: 9." Specific residues of CTLA-4can be referred to as, for example, "CTLA-4 (25)," with reference to SEQ ID NO: 9 implicit.

Accordingly, provided herein are inhibitors of these regulators of B cell-mediated immunosuppression. Such inhibitors are used in the therapeutic compositions or methods of treatment described herein to inhibit TIGIT, PD-1, TIM-3, LAG-3, or CTLA-4 activity or expression in B cells. As used herein, a "regulator of B cell-mediated immunosuppression" refers to any molecule, such as TIGIT, PD-1, TIM-3, LAG-3, and CTLA-4, the expression or activity of which in B cells is required for their steady-state or induced regulatory activities, including inhibition of activated T cells, production of IL-10, maintenance of regulatory Foxp3+ Tregs, etc. As used herein, an "inhibitory agent" or "inhibitor," is an agent that reduces the level and/or activity of a target as compared to the level and/or activity in the absence of the agent by a statistically significant amount. For the avoidance of doubt, reduction by at least 10%, at least 20%, at least 30%, at least 40%, at least 50%, at least 60%, at least 70%, at least 80%, at least 90%, at least 95% or more would be considered inhibitory. The level of a target can be the level of a polypeptide target, the level of an isoform or variant of a polypeptide target, or the level of an RNA transcript encoding the polypeptide target. The activity of a polypeptide target can include, but is not limited to, the ability of the target to bind a normal ligand, the ability of the target to interact with other polypeptides (e.g. downstream signaling partners), and/or the ability of the target polypeptide to effect a downstream response (e.g. phosphorylation levels or gene expression). In some aspects, such inhibitors include inhibitors of TIGIT, PD-1, TIM-3, LAG-3, or CTLA-4 expression or activity in B cells.

Inhibitors of regulators of B cell-mediated immunosuppression can include, by way of non-limiting examples, a protein, an antibody, an antibody reagent which binds specifically to the target, such as TIGIT, PD-1, TIM-3, LAG-3, or CTLA-4, and inhibits its signaling activity, or a small interfering RNA specific for or targeted to the target-encoding mRNA. In some embodiments, an "inhibitor of TIGIT, PD-1, TIM-3, LAG-3, or CTLA-4 activity or expression in B cells" refers to a protein-binding agent that permits inhibition of TIGIT, PD-1, TIM-3, LAG-3, or CTLA-4. Such agents include, but are not limited to, antibodies ("antibodies" includes antigen-binding portions of antibodies such as epitope- or antigen-binding peptides, paratopes, functional CDRs; recombinant antibodies; chimeric antibodies; tribodies; midibodies; or antigen-binding derivatives, analogs, variants, portions, or fragments thereof), multi-specific protein-binding agents, protein-binding agents, small molecules, recombinant protein, peptides, aptamers, avimers and protein-binding derivatives, portions or fragments thereof. In some embodiments, antisense oligonucleotides represent another class of agents that are useful in the compositions and methods described herein. This class of agents and methods for preparing and using them are all well-known in the art, as are ribozyme and miRNA molecules. See, e.g., PCT US2007/024067 for a thorough discussion.

In some embodiments, an inhibitory agent can comprise an antibody or antigen-binding fragment thereof. As used herein, the term "antibody reagent" refers to a polypeptide that includes at least one immunoglobulin variable domain or immunoglobulin variable domain sequence and which specifically binds a given antigen (e.g., TIGIT, PD-1, TIM-3, LAG-3, and/or CTLA-4). In some embodiments, an antibody or antigen-binding fragment thereof can inhibit the signaling activity of the target molecule, e.g., inhibit signaling activity TIGIT, PD-1, TIM-3, LAG-3, and/or CTLA-4.

In some embodiments of the aspects described herein, a polypeptide agent can be formatted as a bispecific polypeptide agent as described herein, and in US 2010/0081796 and US 2010/0021473. In other embodiments of the aspects described herein, a polypeptide agent can be formatted as a multispecific polypeptide agent, for example as described in WO 03/002609.

Bispecific and multispecific polypeptide agents can comprise immunoglobulin variable domains that have different binding specificities. Such bispecific and multispecific polypeptide agents can comprise combinations of heavy and light chain domains. For example, a bispecific polypeptide agent can comprise a V_{H} domain and a V_{L} domain, which can be linked together in the form of an scFv *(e.g.,* using a suitable linker such as Gly₄Ser) that binds one target, *i.e.,* TIGIT, PD-1, TIM-3, LAG-3, or CTLA-4. A construct that includes, e.g., an scFv that binds TIGIT and an scFv that binds a B-cell specific receptor, is said to be bispecific for PD-1 and TIM-3. Similar arrangements can be applied in the context of, *e.g.,* a bispecific F(ab')₂ construct.

Single domain antibody constructs are also contemplated for the development of bispecific reagents described herein. In some embodiments of the aspects described herein, the bispecific and multispecific polypeptide agents may not comprise complementary V_{H}/V_{L} pairs which form an antigen-binding site that binds to a single antigen or epitope co-operatively as found in conventional two chain antibodies. Instead, in some embodiments, the bispecific and multispecific polypeptide agents can comprise a V_{H}/V_{L} complementary pair, wherein the V domains each have different binding specificities, such that two different epitopes or antigens are specifically bound.

In addition, in some embodiments, the bispecific and multispecific polypeptide agents comprise one or more C_{H} or C_{L} domains. A hinge region domain can also be included in some embodiments. Such combinations of domains can, for example, mimic natural antibodies, such as IgG or IgM, or fragments thereof, such as Fv, scFv, Fab or F(ab') ₂ molecules. Other structures, such as a single arm of an IgG molecule comprising V_{H}, V_{L}, C_{H}1 and C_{L} domains, are also encompassed within the embodiments described herein. Alternatively, in another embodiment, a pluralityof bispecific polypeptide agents are combined to form a multimer. For example, two different bispecific polypeptide agents can be combined to create a tetra-specific molecule. It will be appreciated by one skilled in the art that the light and heavy variable regions of a bispecific or multispecific polypeptide agent produced according to the methods described herein can be on the same polypeptide chain, or alternatively, on different polypeptide chains. In the case where the variable regions are on different polypeptide chains, then they can be linked via a linker, generally a flexible linker (such as a polypeptide chain), a chemical linking group, or any other method known in the art.

In different embodiments of the aspects described herein, the bispecific and multispecific polypeptide agents can be formatted as bi- or multispecific antibodies or antigen-binding fragments thereof, or into bi- or multispecific non-antibody structures. Suitable formats include, for example, any suitable polypeptide structure in which an antibody variable domain, or one or more of the CDRs thereof, can be incorporated so as to confer binding specificity for antigen on the structure. A variety of suitable antibody formats are known in the art, such as, bispecific IgG-like formats (*e.g*., chimeric antibodies, humanized antibodies, human antibodies, single chain antibodies, heterodimers of antibody heavy chains and/or light chains, antigen-binding fragments of any of the foregoing (e.g., a Fv fragment (e.g., single chain Fv (scFv), a disulfide bonded Fv), a Fab fragment, a Fab' fragment, a F(ab')₂ fragment), a single variable domain (e.g., V_{H}, V_{L}, V_{HH}), a dAb, and modified versions of any of the foregoing *(e.g.,* modified by the covalent attachment of polyalkylene glycol *(e.g.,* polyethylene glycol, polypropylene glycol, polybutylene glycol) or other suitable polymer).

A bispecific or multispecific polypeptide agent can be formatted using a suitable linker such as (Gly₄Ser)ₙ, where n=from 1 to 8, *e.g.,* 2, 3, 4, 5, 6 or 7. If desired, bispecific or multispecific polypeptide agents can be linked to an antibody Fc region, comprising one or both of C_{H}2 and C_{H}3 domains, and optionally a hinge region. For example, vectors encoding bispecific or multispecific polypeptide agents linked as a single nucleotide sequence to an Fc region can be used to prepare such polypeptides.

In some embodiments of the aspects described herein, antigen-binding fragments of antibodies can be combined and/or formatted into non-antibody multispecific polypeptide structures to form multivalent complexes, which bind target molecules having the same epitope, thereby providing superior avidity. For example, natural bacterial receptors such as SpA can been used as scaffolds for the grafting of CDRs to generate ligands which bind specifically to one or more epitopes. Details of this procedure are described in U.S. Pat. No. 5,831,012. Other suitable scaffolds include those based on fibronectin and affibodies. Details of suitable procedures are described in WO 98/58965. Other suitable scaffolds are described in van den Beuken et al., J. Mol. Biol. 310:591-601 (2001), and scaffolds such as those described in WO 00/69907 (Medical Research Council) which are based for example on the ring structure of bacterial GroEL or other chaperone polypeptides. In some embodiments, protein scaffolds can be combined.

In some embodiments of the aspects described herein, the bispecific or multispecific polypeptide agents can be formatted as fusion proteins that contain a first antigen-binding domain that is fused directly to a second antigen-binding domain. If desired, in some embodiments, such a format can further comprise a half-life extending moiety. For example, the bispecific or multispecific polypeptide agent can comprise a first antigen-binding domain specific for TIGIT, that is fused directly to a second antigen-binding domain specific for TIM-3, that is fused directly to an antigen-binding domain that binds serum albumin.

Generally, the orientation of the polypeptide domains that have a binding site with binding specificity for a target, and whether a bispecific or multispecific polypeptide agent comprises a linker, are a matter of design choice. However, some orientations, with or without linkers, can provide better binding characteristics than other orientations. All orientations are encompassed by the aspects and embodiments described herein, and bispecific or multispecific polypeptide agents that contain an orientation that provides desired binding characteristics can be easily identified by screening.

In some embodiments of the aspects described herein, an inhibitor targets both TIGIT, PD-1, TIM-3, LAG-3, and/or CTLA-4 and a B cell-specific cell surface receptor, in order to inhibit or reduce expression or activity of TIGIT, PD-1, TIM-3, LAG-3, and/or CTLA-4 specifically in B cells. As used herein, a "B cell-specific cell surface receptor" refers to a molecule found on the surface of B cells that is not expressed or expressed minimally in other cell populations, such as T cells. Non-limiting examples of B cell-specific surface receptors useful in the compositions and methods described herein include CD19, CD20, and CD22. Non-limiting examples of therapeutic antibodies that can be used to generate the multispecific agents comprising at least one binding domain that binds to a B cell-specific cell-surface molecule include Rituximab (anti-CD20), Ofatumumab (anti-CD20), Ocrelizumab (anti-CD20), Veltuzumab (anti-CD20), MEDI-551 (anti-CD19), and Epratuzumab (anti-CD22).

Accordingly, in some aspects, described herein are multispecific agents comprising at least one binding domain that specifically binds to a B cell-specific regulator, such as TIGIT, PD-1, TIM-3, LAG-3, and/or CTLA-4, and at least one binding domain that specifically binds to a B cell-specific cell-surface molecule.

In some aspects, described herein are bispecific agents comprising at least one binding domain that specifically binds to a B cell-specific regulator, such as TIGIT, PD-1, TIM-3, LAG-3, or CTLA-4, and at least one binding domain that specifically binds to a B cell-specific cell-surface molecule.

It is to be understood that the bispecific or multispecific polypeptide agents described herein will generally bind to naturally occurring or synthetic analogs, variants, mutants, alleles, parts and fragments of a TIGIT, PD-1, TIM-3, LAG-3, or CTLA-4 target; or at least to those analogs, variants, mutants, alleles, parts and fragments of a TIGIT, PD-1, TIM-3, LAG-3, or CTLA-4 target, that contain one or more antigenic determinants or epitopes that are essentially the same as the antigenic determinant(s) or epitope(s) to which the bispecific or multispecific polypeptide agents described herein bind on the TIGIT, PD-1, TIM-3, LAG-3, or CTLA-4 target. In some embodiments, the amino acid sequences and polypeptides described herein bind to some analogs, variants, mutants, alleles, parts and fragments of a TIGIT, PD-1, TIM-3, LAG-3, or CTLA-4 target, but not to others.

In some embodiments of the aspects described herein, the binding sites of the bispecific polypeptide agents, such as the bispecific antibodies, are directed against a target's ligand interaction site. In other embodiments of the aspects described herein, the binding sites of the bispecific polypeptide agents are directed against a site on a target in the proximity of the ligand interaction site, in order to provide steric hindrance for the interaction of the target with its receptor or ligand. Preferably, the site against which the bispecific polypeptide agents described herein are directed is such that binding of the target to its receptor or ligand is modulated, and in particular, inhibited or prevented.

Antibodies suitable for practicing the methods described herein are preferably monoclonal and multispecific, and can include, but are not limited to, human, humanized or chimeric antibodies, comprising single chain antibodies, Fab fragments, F(ab') fragments, fragments produced by a Fab expression library, and/or binding fragments of any of the above. Antibodies also refer to immunoglobulin molecules and immunologically active portions of immunoglobulin molecules, *i*.*e*., molecules that contain at least two antigen or target binding sites that specifically bind PD-1 and TIM-3. The immunoglobulin molecules described herein can be of any type *(e.g.,* IgG, IgE, IgM, IgD, IgA and IgY), class *(e.g.,* IgG1, IgG2, IgG3, IgG4, IgA1 and IgA2) or subclass of immunoglobulin molecule, as is understood by one of skill in the art.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring production of the antibody by any particular method. For example, the monoclonal antibodies to be used in accordance with the invention may be made by the hybridoma method first described by Kohler et al., Nature 256:495 (1975), or may be made by recombinant DNA methods (see, *e.g.,* U.S. Pat. No. 4,816,567). The "monoclonal antibodies" may also be isolated from phage antibody libraries using the techniques described in Clackson et al., Nature 352:624-628 (1991) or Marks et al., J. Mol. Biol. 222:581-597 (1991), for example.

The term "antibody fragment," as used herein, refer to a protein fragment that comprises only a portion of an intact antibody, generally including an antigen binding site of the intact antibody and thus retaining the ability to bind antigen. Examples of antibody fragments encompassed by the present definition include: (i) the Fab fragment, having V_{L}, C_{L}, V_{H} and C_{H}1 domains; (ii) the Fab' fragment, which is a Fab fragment having one or more cysteine residues at the C-terminus of the C_{H}1 domain; (iii) the Fd fragment having V_{H} and C_{H}1 domains; (iv) the Fd' fragment having V_{H} and C_{H}1 domains and one or more cysteine residues at the C-terminus of the CH1 domain; (v) the Fv fragment having the V_{L} and V_{H} domains of a single arm of an antibody; (vi) the dAb fragment (Ward et al., Nature 341, 544-546 (1989)) which consists of a V_{H} domain; (vii) isolated CDR regions; (viii) F(ab')₂ fragments, a bivalent fragment including two Fab' fragments linked by a disulphide bridge at the hinge region; (ix) single chain antibody molecules *(e.g.* single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (V_{H}) connected to a light chain variable domain (V_{L}) in the same polypeptide chain (see, *e.g.,* EP 404,097; WO 93/11161; and Hollinger et al., Proc. Natl. Acad. Sci. USA, 90:6444-6448 (1993)); (xi) "linear antibodies" comprising a pair of tandem Fd segments (V_{H}-C_{H}1-V_{H}-C_{H}1) which, together with complementary light chain polypeptides, form a pair of antigen binding regions (Zapata et al. Protein Eng. 8(10): 1057-1062 (1995); and U.S. Pat. No. 5,641,870).

In another aspect, bispecific antibodies having an IgG-like format are provided. Such formats have the conventional four chain structure of an IgG molecule (2 heavy chains and two light chains), in which one antigen-binding region (comprised of a V_{H} and a V_{L} domain) specifically binds TIGIT, PD-1, TIM-3, LAG-3, or CTLA-4 and the other antigen-binding region (also comprised of a V_{H} and a V_{L} domain) specifically binds a B cells-specific receptor. In some embodiments, each of the variable regions (2 V_{H} regions and 2 V_{L} regions) is replaced with a dAb or single variable domain. The dAb(s) or single variable domain(s) that are included in an IgG-like format can have the same specificity or different specificities. In some embodiments, the IgG-like format is tetravalent and can have two, three or four specificities. For example, the IgG-like format can be bispecific and comprise 3 dAbs that have the same specificity and another dAb that has a different specificity; bispecific and comprise two dAbs that have the same specificity and two dAbs that have a common but different specificity; trispecific and comprise first and second dAbs that have the same specificity, a third dAb with a different specificity and a fourth dAb with a different specificity from the first, second and third dAbs; or tetraspecific and comprise four dAbs that each have a different specificity. Antigen-binding fragments of IgG-like formats (e.g., Fab, F(ab')₂, Fab', Fv, scFv) can be prepared as is known to one of skill in the art, and as described herein.

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule is usually done by affinity chromatography steps, but the product yields are low. Similar procedures are disclosed in WO 93/08829, and in Traunecker et al, EMBO J., 10:3655-3659 (1991).

According to another approach, described in WO96/27011, the interface between a pair of antibody molecules can be engineered to maximize the percentage of heterodimers which are recovered from recombinant cell culture. Such interfaces can comprise at least a part of the CH₃ domain of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

In one aspect, the bispecific antibodies described herein include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/200373, and EP 03089). Heteroconjugate antibodies can be made using any convenient cross-linking methods. Suitable cross-linking agents are well known in the art, and are disclosed in U.S. Pat. No. 4,676,980, along with a number of cross-linking techniques. In one embodiment, the bispecific antibodies do not comprise a heteroconjugate.

Techniques for generating bispecific antibodies from antibody fragments have also been described in the literature. For example, bispecific antibodies can be prepared using chemical linkage. For example, Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoet-hylamine and is mixed with an equimolar amount of the other Fab'-TNB derivative to form the bispecific antibody. A bispecific antibody produced using this method can be used in any of the compositions and methods described herein.

In some embodiments, bispecific antibodies for use in the compositions and methods described herein can be produced using any of the methods described in U.S. Patent Application No.: 20100233173; U.S. Patent Application No.: 20100105873; U.S. Patent Application No.: 20090155275; U.S. Patent Application No.: 20080071063; and U.S. Patent Application No.: 20060121042, the contents of each of which are herein incoporated in their entireties by reference. In some embodiments, a bispecific antibody specific for PD-1 and TIM-3 can be produced using any of the methods described in U.S. Patent Application No.: 20090175867 and U.S. Patent Application No.: 20110033483the contents of which are herein incoporated in their entireties by reference.

In some embodiments, the bispecific antibodies can be made by the direct recovery of Fab'-SH fragments recombinantly expressed, *e.g.,* in E. *coli,* and can be chemically coupled to form bispecific antibodies. For example, Shalaby et al., J Exp. Med, 175: 217-225 (1992) describe the production of a fully humanized bispecific antibody F(ab')₂ molecule. Each Fab' fragment was separately secreted from E. *coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described, and can be used in the generation of the bispecific antibodies. For example, bispecific antibodies have been produced using leucine zippers (Kostelny et al., J. Immunol, 148(5):1547-1553 (1992)). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{H} and V_{L} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994). Alternatively, the antibodies can be "linear antibodies" as described in Zapata et al. Protein Eng. 8(10):1057-1062 (1995). Briefly, these antibodies comprise a pair of tandem Fd segments (V_{H}-C_{H}1-V_{H}-C _{H}1) which form a pair of antigen binding regions. Linear antibodies can be bispecific or multispecific.

Antibodies useful in the present methods can be described or specified in terms of the particular CDRs they comprise. The compositions and methods described herein encompass the use of an antibody or derivative thereof comprising a heavy or light chain variable domain, where the variable domain comprises (a) a set of three CDRs, and (b) a set of four framework regions, and in which the antibody or antibody derivative thereof specifically binds TIGIT, PD-1, TIM-3, LAG-3, or CTLA-4.

Also provided herein are chimeric antibody derivatives of the bispecific and mutispecific polypeptide agents, *i.e.,* antibody molecules in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)). Chimeric antibody molecules can include, for example, one or more antigen binding domains from an antibody of a mouse, rat, or other species, with human constant regions. A variety of approaches for making chimeric antibodies have been described and can be used to make chimeric antibodies containing the immunoglobulin variable region which recognizes the selected antigens, *i.e.,* TIGIT, PD-1, TIM-3, LAG-3, or CTLA-4, on the surface of differentiated cells or tumor-specific cells. See, for example, Takeda et al., 1985, Nature 314:452; Cabilly et al., U.S. Pat. No. 4,816,567; Boss *et al.*,; Tanaguchi et al., European Patent Publication EP171496; European Patent Publication 0173494, United Kingdom patent GB 2177096B).

The bispecific and mutispecific polypeptide agents described herein can also be a humanized antibody derivative. Humanized forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

Chemical conjugation can also be used to generate the bispecific or multispecific antibodies described herein, and is based on the use of homo- and heterobifunctional reagents with E-amino groups or hinge region thiol groups. Homobifunctional reagents such as 5,5'-Dithiobis(2-nitrobenzoic acid) (DNTB) generate disulfide bonds between the two Fabs, and 0-phenylenedimaleimide (O-PDM) generate thioether bonds between the two Fabs (Brenner *et al. ,* 1985, Glennie *et al.,* 1987). Heterobifunctional reagents such as N-succinimidyl-3-(2-pyridylditio) propionate (SPDP) combine exposed amino groups of antibodies and Fab fragments, regardless of class or isotype (Van Dijk *et al.,* 1989).

In some embodiments, the antibodies described herein include derivatives that are modified, *i.e.,* by the covalent attachment of any type of molecule to the antibody such that covalent attachment does not prevent the antibody from binding to TIGIT, PD-1, TIM-3, LAG-3, or CTLA-4. For example, but not by way of limitation, the antibody derivatives include antibodies that have been modified, *e.g.,* by glycosylation, acetylation, pegylation, phosphorylation, amidation, derivatization by known protecting/blocking groups, proteolytic cleavage, linkage to a cellular ligand or other protein, etc. Any of numerous chemical modifications can be carried out by known techniques, including, but not limited to specific chemical cleavage, acetylation, formylation, metabolic synthesis of turicamycin, etc. Additionally, the derivative can contain one or more non-classical amino acids.

Accordingly, the bispecific or multispecific antibodies described herein can be generated by any suitable method known in the art. Monoclonal and polyclonal antibodies against TIGIT, PD-1, TIM-3, LAG-3, or CTLA-4 are known in the art. To the extent necessary, e.g., to generate antibodies with particular characteristics or epitope specificity, the skilled artisan can generate new monoclonal or polyclonal anti-PD-1 and anti-TIM-3 antibodies as discussed below or as known in the art. In other embodiments, the bispecific and multispecific antibodies and antigen-binding fragments thereof described herein can utilize PD-1 binding site sequences from monoclonal antibodies against human PD-1, such as, MDX-1106 (ONO-4538), a fully human IgG4 anti-PD-1 blocking antibody (Journal of Clinical Oncology, 2008 Vol 26, No 15S); CT-011 (CureTech, LTD, previously CT-AcTibody or BAT), a humanized monoclonal IgG1 antibody (Benson DM et al., Blood. 2010 May 11), or those obtained from, clone NAT (Abcam), clone EH12.2H7 (Biolegend), clone J116 (eBioscience), clone MIH4 (eBioscience), clone J105 (eBioscience), or clone 192106 (R& D systems). Similarly, the bispecific and multispecific antibodies and antigen-binding fragments thereof described herein can utilize TIM-3 binding site sequences from monoclonal antibodies against human TIM-3, such as those obtained from, clone F38-2E2 (Biolegend), or clone 344823 (R&D Systems). For example, an antigen binding site against PD-1 having the amino acid sequences of the CDR regions of MDX-1106, and an antigen binding site against TIM-3 having the amino acid sequences of the CDR regions of the antibody produced by clone 344823 can be grafted onto an appropriate framework, such as a human IgG1 backbone, to generate a bispecific antibody construct as described herein.

Monoclonal antibodies can be prepared using a wide variety of techniques known in the art including the use of hybridoma, recombinant, and phage display technologies, or a combination thereof. Various methods for making monoclonal antibodies described herein are available in the art. For example, the monoclonal antibodies can be made using the hybridoma method first described by Kohler et al., Nature, 256:495 (1975), or by recombinant DNA methods (U.S. Pat. No. 4,816,567). For example, monoclonal antibodies can be produced using hybridoma techniques including those known in the art and taught, for example, in Harlow et al., Antibodies: A Laboratory Manual, (Cold Spring Harbor Laboratory Press, 2nd ed., 1988); Hammer-ling, et al., in: Monoclonal Antibodies and T-Cell Hybrido-mas 563-681 (Elsevier, N.Y., 1981). The term "monoclonal antibody" as used herein is not limited to antibodies produced through hybridoma technology. It is to be understood that the term "monoclonal antibody" refers to an antibody that is derived from a single clone, including any eukaryotic, prokaryotic, or phage clone, and not the method by which it is produced.

In some aspects, provided herein methods of reducing B-cell-mediated immunosuppression comprising administering an inhibitor of TIGIT, PD-1, TIM-3, LAG-3, or CTLA-4 activity or expression in B cells to a subject in need thereof.

In some embodiments of these aspects and all such aspects described herein, the subject in need thereof has or has been diagnosed with cancer.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

Metastases are most often detected through the sole or combined use of magnetic resonance imaging (MRI) scans, computed tomography (CT) scans, blood and platelet counts, liver function studies, chest X-rays and bone scans in addition to the monitoring of specific symptoms.

Examples of cancer include but are not limited to, carcinoma, lymphoma, blastoma, sarcoma, and leukemia. More particular examples of such cancers include, but are not limited to basal cell carcinoma, biliary tract cancer; bladder cancer; bone cancer; brain and CNS cancer; breast cancer; cancer of the peritoneum; cervical cancer; cholangiocarcinoma; choriocarcinoma; colon and rectum cancer; connective tissue cancer; cancer of the digestive system; endometrial cancer; esophageal cancer; eye cancer; cancer of the head and neck; gastric cancer (including gastrointestinal cancer); glioblastoma; hepatic carcinoma; hepatoma; intra-epithelial neoplasm; kidney or renal cancer; larynx cancer; leukemia; liver cancer; lung cancer (e.g., small-cell lung cancer, non-small cell lung cancer, adenocarcinoma of the lung, and squamous carcinoma of the lung); lymphoma including Hodgkin's and non-Hodgkin's lymphoma; melanoma; myeloma; neuroblastoma; oral cavity cancer (e.g., lip, tongue, mouth, and pharynx); ovarian cancer; pancreatic cancer; prostate cancer; retinoblastoma; rhabdomyosarcoma; rectal cancer; cancer of the respiratory system; salivary gland carcinoma; sarcoma; skin cancer; squamous cell cancer; stomach cancer; teratocarcinoma; testicular cancer; thyroid cancer; uterine or endometrial cancer; cancer of the urinary system; vulval cancer; as well as other carcinomas and sarcomas; as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), tumors of primitive origins and Meigs' syndrome.

In some embodiments of these methods and all such methods described herein, the methods further comprise admininstering an anti-cancer therapy or agent to a subject in addition to the inhibitor of TIGIT, PD-1, TIM-3, LAG-3, or CTLA-4 activity or expression in B cells

The term "anti-cancer therapy" refers to a therapy useful in treating cancer. Examples of anti-cancer therapeutic agents include, but are not limited to, e.g., surgery, chemotherapeutic agents, growth inhibitory agents, cytotoxic agents, agents used in radiation therapy, anti-angiogenesis agents, apoptotic agents, anti-tubulin agents, and other agents to treat cancer, such as anti-HER2 antibodies (e.g., HERCEPTIN^{®}), anti-CD20 antibodies, an epidermal growth factor receptor (EGFR) antagonist *(e.g.,* a tyrosine kinase inhibitor), HER1/EGFR inhibitor *(e.g.,* erlotinib (TARCEVA^{®})), platelet derived growth factor inhibitors *(e.g.,* GLEEVEC^{™} (Imatinib Mesylate)), a COX2 inhibitor *(e.g.,* celecoxib), interferons, cytokines, antagonists (e.g., neutralizing antibodies) that bind to one or more of the following targets PD1, PDL1, PDL2, TIM3 or any TIM family member, CEACAM1 or any CEACAM family member, ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also specifically contemplated for the methods described herein.

In some embodiments, an anti-cancer therapy comprises an immunotherapy such as adoptive cell transfer. "Adoptive cell transfer," as used herein, includes immunotherapies involving genetically engineering a subject or patient's own T cells to produce special receptors on their surface called chimeric antigen receptors (CARs). CARs are proteins that allow the T cells to recognize a specific protein (antigen) on tumor cells. These engineered CAR T cells are then grown in the laboratory until they number in the billions. The expanded population of CAR T cells is then infused into the patient. After the infusion, the T cells multiply in the subject's body and, with guidance from their engineered receptor, recognize and kill cancer cells that harbor the antigen on their surfaces.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including active fragments and/or variants thereof.

In some embodiments of these methods and all such methods described herein, the methods further comprise admininstering a chemotherapeutic agent to the subject being administered the inhibitor of TIGIT, PD-1, TIM-3, LAG-3, or CTLA-4 activity or expression in B cells

Non-limiting examples of chemotherapeutic agents can include include alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (see, e.g., Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol-Myers Squibb Oncology, Princeton, N.J.), ABRAXANE^{®} Cremophor-free, albumin-engineered nanoparticle formulation of paclitaxel (American Pharmaceutical Partners, Schaumberg, Ill.), and TAXOTERE^{®} doxetaxel (Rhone-Poulenc Rorer, Antony, France); chloranbucil; GEMZAR^{®} gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE, vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylomithine (DMFO); retinoids such as retinoic acid; capecitabine; combretastatin; leucovorin (LV); oxaliplatin, including the oxaliplatin treatment regimen (FOLFOX); lapatinib (TYKERB.); inhibitors of PKC-alpha, Raf, H-Ras, EGFR (e.g., erlotinib (TARCEVA^{®})) and VEGF-A that reduce cell proliferation and pharmaceutically acceptable salts, acids or derivatives of any of the above. In addition, the methods of treatment can further include the use of radiation or radiation therapy.

As used herein, the terms "chemotherapy" or "chemotherapeutic agent" refer to any chemical agent with therapeutic usefulness in the treatment of diseases characterized by abnormal cell growth. Such diseases include tumors, neoplasms and cancer as well as diseases characterized by hyperplastic growth. Chemotherapeutic agents as used herein encompass both chemical and biological agents. These agents function to inhibit a cellular activity upon which the cancer cell depends for continued survival. Categories of chemotherapeutic agents include alkylating/alkaloid agents, antimetabolites, hormones or hormone analogs, and miscellaneous antineoplastic drugs. Most if not all of these agents are directly toxic to cancer cells and do not require immune stimulation. In one embodiment, a chemotherapeutic agent is an agent of use in treating neoplasms such as solid tumors. In one embodiment, a chemotherapeutic agent is a radioactive molecule. One of skill in the art can readily identify a chemotherapeutic agent of use (*e.g.* see Physicians' Cancer Chemotherapy Drug Manual 2014, Edward Chu, Vincent T. DeVita Jr., Jones & Bartlett Learning; Principles of Cancer Therapy, Chapter 85 in Harrison's Principles of Internal Medicine, 18th edition; Therapeutic Targeting of Cancer Cells: Era of Molecularly Targeted Agents and Cancer Pharmacology, Chs. 28-29 in Abeloff's Clinical Oncology, 2013 Elsevier; Baltzer L, Berkery R (eds): Oncology Pocket Guide to Chemotherapy, 2nd ed. St. Louis, Mosby-Year Book, 1995; Fischer D S (ed): The Cancer Chemotherapy Handbook, 4th ed. St. Louis, Mosby-Year Book, 2003)).

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

In some embodiments of these methods and all such methods described herein, the methods further comprise admininstering a tumor or cancer antigen to a subject being administered inhibitor of TIGIT, PD-1, TIM-3, LAG-3, or CTLA-4 activity or expression in B cells.

A number of tumor antigens have been identified that are associated with specific cancers. As used herein, the terms "tumor antigen" and "cancer antigen" are used interchangeably to refer to antigens which are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent *(i.e.,* not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), and fusion proteins resulting from internal deletions or chromosomal translocations. Still other cancer antigens can be encoded by viral genes such as those carried on RNA and DNA tumor viruses. Many tumor antigens have been defined in terms of multiple solid tumors: MAGE 1, 2, & 3, defined by immunity; MART-1/Melan-A, gp100, carcinoembryonic antigen (CEA), HER2, mucins *(i.e.,* MUC-1), prostate-specific antigen (PSA), and prostatic acid phosphatase (PAP). In addition, viral proteins such as hepatitis B (HBV), Epstein-Barr (EBV), and human papilloma (HPV) have been shown to be important in the development of hepatocellular carcinoma, lymphoma, and cervical cancer, respectively. However, due to the immunosuppression of patients diagnosed with cancer, the immune systems of these patients often fail to respond to the tumor antigens.

By "reduce" or "inhibit" in terms of the cancer treatment methods described herein is meant the ability to cause an overall decrease preferably of 20% or greater, 30% or greater, 40% or greater, 45% or greater, more preferably of 50% or greater, of 55% or greater, of 60 % or greater, of 65% or greater, of 70% or greater, and most preferably of 75% or greater, 80% or greater, 85% or greater, 90% or greater, or 95% or greater, for a given parameter or symptom. Reduce or inhibit can refer to, for example, the symptoms of the disorder being treated, the presence or size of metastases or micrometastases, the size of the primary tumor, the presence or the size of the dormant tumor, etc.

As used herein, "alleviating a symptom of a cancer or tumor" is ameliorating any condition or symptom associated with the cancer such as the symptoms of the cancer being treated, the presence or size of metastases or micrometastases, the size of the primary tumor, the presence or the size of the dormant tumor, etc. As compared with an equivalent untreated control, such as a subject prior to the administration of the inhibitors described herein, such reduction or degree of prevention is at least 5%, 10%, 20%, 40%, 50%, 60%, 80%, 90%, 95%, or more as measured by any standard technique known to one of ordinary skill in the art. A patient or subject who is being treated for a cancer or tumor is one who a medical practitioner has diagnosed as having such a condition. Diagnosis can be by any suitable means.

Also provided herein, in some aspects, are methods of treating a disease or disorder involving inappropriate immunosuppression, the method comprising administering a therapeutically effective amount of an agent that increases the expression or activity of TIGIT, PD-1, TIM-3, LAG-3, or CTLA-4 in B cells to a subject in need thereof.

In some embodiments of these methods and all such methods described herein, a subject in need thereof has or has been diagnosed with an autoimmune disease or disorder.

Accordingly, in some embodiments of these methods and all such methods described herein, the autoimmune diseases to be treated or prevented using the methods described herein, include, but are not limited to: rheumatoid arthritis, Crohn's disease or colitis, multiple sclerosis, systemic lupus erythematosus (SLE), autoimmune encephalomyelitis, myasthenia gravis (MG), Hashimoto's thyroiditis, Goodpasture's syndrome, pemphigus (e.g., pemphigus vulgaris), Grave's disease, autoimmune hemolytic anemia, autoimmune thrombocytopenic purpura, scleroderma with anti-collagen antibodies, mixed connective tissue disease, polymyositis, pernicious anemia, idiopathic Addison's disease, autoimmune- associated infertility, glomerulonephritis (e.g., crescentic glomerulonephritis, proliferative glomerulonephritis), bullous pemphigoid, Sjogren's syndrome, insulin resistance, and autoimmune diabetes mellitus (type 1 diabetes mellitus; insulin- dependent diabetes mellitus). Autoimmune disease has been recognized also to encompass atherosclerosis and Alzheimer's disease. In some embodiments of the aspects described herein, the autoimmune disease is selected from the group consisting of multiple sclerosis, type-I diabetes, Hashimoto's thyroiditis, Crohn's disease or colitis, rheumatoid arthritis, systemic lupus erythematosus, gastritis, autoimmune hepatitis, hemolytic anemia, autoimmune hemophilia, autoimmune lymphoproliferative syndrome (ALPS), autoimmune uveoretinitis, glomerulonephritis, Guillain-Barre syndrome, psoriasis and myasthenia gravis.

In some embodiments of these methods and all such methods described herein, a subject in need thereof has or has been diagnosed with host versus graft disease (HVGD). In a further such embodiment, the subject being treated with the methods described herein is an organ or tissue transplant recipient. In some embodiments, the methods are used for increasing transplantation tolerance in a subject. In some such embodiments, the subject is a recipient of an allogenic transplant.

The transplant can be any organ or tissue transplant, including but not limited to heart, kidney, liver, skin, pancreas, bone marrow, skin or cartilage. "Transplantation tolerance," as used herein, refers to a lack of rejection of the donor organ by the recipient's immune system.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a disease or disorder. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to, alleviation of one or more symptom(s), diminishment of extent of disease, stabilized (*i.e.,* not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment).

The terms "subject," "patient," and "individual" as used in regard to any of the methods described herein are used interchangeably herein, and refer to an animal, for example a human, recipient of the inhibitos described herein. For treatment of disease states which are specific for a specific animal such as a human subject, the term "subject" refers to that specific animal. The terms "non-human animals" and "non-human mammals" are used interchangeably herein, and include mammals such as rats, mice, rabbits, sheep, cats, dogs, cows, pigs, and non-human primates. The term "subject" also encompasses any vertebrate including but not limited to mammals, reptiles, amphibians and fish. However, advantageously, the subject is a mammal such as a human, or other mammals such as a domesticated mammal, *e.g.* dog, cat, horse, and the like. Production mammal, *e.g.* cow, sheep, pig, and the like are also encompassed in the term subject.

The term "effective amount" as used herein refers to the amount of any of the inhibitors or agents described herein needed to alleviate at least one or more symptom of the disease or disorder being treated, and relates to a sufficient amount of pharmacological composition to provide the desired effect. The term "therapeutically effective amount" therefore refers to an amount of the inhibitors or potentiators described herein, using the methods as disclosed herein, that is sufficient to provide a particular effect when administered to a typical subject. An effective amount as used herein would also include an amount sufficient to delay the development of a symptom of the disease, alter the course of a symptom disease (for example but not limited to, slow the progression of a symptom of the disease), or reverse a symptom of the disease. Thus, it is not possible to specify the exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. Compositions, methods, and uses that exhibit large therapeutic indices are preferred. A therapeutically effective dose can be estimated initially from cell culture assays. Also, a dose can be formulated in animal models to achieve a circulating plasma concentration range that includes the IC50, which achieves a half-maximal inhibition of measured function or activity) as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

The agents described herein can be administered to a subject in need thereof by any appropriate route which results in an effective treatment in the subject. As used herein, the terms "administering," and "introducing" are used interchangeably and refer to the placement of an agent into a subject by a method or route which results in at least partial localization of such agents at a desired site, such as a tumor site or site of inflammation, such that a desired effect(s) is produced.

In some embodiments, the agents described herein can be administered to a subject by any mode of administration that delivers the agent systemically or to a desired surface or target, and can include, but is not limited to, injection, infusion, instillation, and inhalation administration. To the extent that polypeptide agents can be protected from inactivation in the gut, oral administration forms are also contemplated. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion.

The phrases "parenteral administration" and "administered parenterally" as used herein, refer to modes of administration other than enteral and topical administration, usually by injection. The phrases "systemic administration," "administered systemically", "peripheral administration" and "administered peripherally" as used herein refer to the administration of the agents described herein, other than directly into a target site, tissue, or organ, such that it enters the subject's circulatory system and, thus, is subject to metabolism and other like processes.

It is understood that the foregoing description and the following examples are illustrative only and are not to be taken as limitations upon the scope of the invention. Various changes and modifications to the disclosed embodiments, which will be apparent to those of skill in the art, may be made without departing from the spirit and scope of the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents are based on the information available to the applicants and do not constitute any admission as to the correctness of the dates or contents of these documents.

These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### EXAMPLES

In addition to producing antibodies for humoral immunity, B cells are also generally considered to act as positive regulators of immune responses by serving as antigen presenting cells (APC) and producing cytokines for optimal T cell activation. However, a subpopulation of B cells, collectively called "regulatory B cells" ("Bregs"), has also been shown to negatively regulate immune responses and thus contribute to the maintenance of tolerance and limitation of inflammation and autoimmunity. Studies have suggested that Bregs exert their regulatory function mainly via an IL-10-dependent manner, but IL-10-independent mechanisms for Breg-mediated suppression of inflammation and autoimmunity are also being increasingly recognized.

In spite of their critical role in regulating immune and autoimmune responses, the heterogeneity of Bregs has hampered our understanding of the critical biologic functions of Bregs. Furthermore, the processes and mechanisms by which Bregs are generated have not been identified.

Tim-1 is a transmembrane glycoprotein expressed in several immune subsets and regulates their responses. It has been shown that a large majority of IL-10 producing B cells are Tim-1 positive (Tim-1+) B cells, regardless of other markers, and that transfer of Tim-1+ B cells inhibit experimental autoimmune encephalomyelitis (EAE), allograft rejection, and allergic airway inflammation. Mice with either global Tim-1 deficiency (Tim-1-/- ) or harboring a loss of function Tim-1 mutant (Tim-1Δmucin ) showed profound defects in B cell IL-10 production, and with age developed severe spontaneous multiorgan tissue inflammation. Their B cells produced more proinflammatory cytokines, and promoted Th1 and Th17 responses, but inhibited the generation of Foxp3+ Tregs and Tr1 cells. Mechanistically, Tim-1 as a phosphatidylserine receptor is required for optimal IL-10 production and function of Bregs by sensing apoptotic cells (AC), and Tim-1 expression in B cells is required for AC treatment-mediated inhibition of EAE. Importantly, it has been demonstrated that B cell IL-10 is enriched in Tim-1+ cells, which can suppress T cell responses, in various models of inflammatory settings and several human diseases. These data strongly indicate that Tim-1 is critical for Breg IL-10 production and immunosuppressive function in tolerance maintenance and inflammation restraint.

However, a causal role for Tim-1 in Breg IL-10 production and immunosuppressive function cannot be firmly established without mice with aTim-1 deficiency only in B cells, as Tim-1 is broadly expressed by various immune and non-immune cells. Accordingly, to clearly and faithfully establish the role of Tim-1 in Bregs and their role in tolerance maintenance and inflammation restraint, as described herein, mice with Tim-1 deficiency only in B cells were generated. It was found that the mice with age developed spontaneous inflammation in multiple organs and tissues, even in brain. While healthy at a young age, the mice developed more severe EAE with increased pathogenic Th1 and Th17 responses and decreased protective IL-10+ and Foxp3+ regulatory T cells.

Furthermore, it was demonstrated that in addition to IL-10, Tim-1+ B cells also highly express a set of co-inhibitory molecules (e.g., TIGIT) and transcription factors (e.g., AhR), whose optimal expression was impaired in the absence of Tim-1 expression signaling. It was found that TIGIT is required for optimal B cell IL-10 production and for Tim-1+ B cell-mediated inhibition of EAE. As demonstrated herein, mice with B cell-specific TIGIT deficiency preferentially developed spontaneous EAE-like disease with inflammation in brain. Moreover, it was shown that AhR is required for Tim-1 regulated B cell expression of IL-10 and TIGIT and for Breg-immunosuppression. Collectively, the data described herein strongly indicate that Tim-1 is functionally critical and essential for generation and maintenance and function of Bregs with multiple regulatory mechanisms, and Tim-1+ Bregs are required for maintaining immune tolerance and limiting inflammation.

### Generation of mice with Tim-1 deficiency only in B cells (Tim-1BKO mice)

To clearly and faithfully demonstrate the role of Tim-1 in Bregs, Tim-1 floxed (Tim-1^{fl/fl}) mice were successfully generated on C57BL/6 background by introducing two Loxp sites into introns 1 and 3 of the Tim1 (Havcr1) gene, respectively, such that Cre-mediated deletion results in excision of exons 2 and 3, encoding the Tim-1 IgV domain and mucin domain (FIG. 1A). CD19Cre/WT Tim-1fl/fl (Tim-1BKO ) mice were generated by crossing Tim-1fl/fl mice and CD19cre mice (a Cre cassette was introduced into exon 2 of the CD19 gene; Homozygous mutant mice are CD19-deficient while heterozygous mice can be used for specific deletion of floxed targets in B cells (43)). Efficient B cell-specific Tim-1 was first confirmed deletion by both flow cytometry (FIG. 1B) and realtime-PCR. While Tim-1 is expressed on WT B cells ex vivo and further increased after anti-CD40 treatment, Tim-1 expression was barely detectable in B cells from Tim-1BKO and Tim-1-/- mice. As shown previously, CD11c+ cells from WT mice expressed Tim-1. While the cells from Tim-1-/- mice lost Tim-1 expression, CD11c+ cells from Tim-1BKO mice still expressed Tim-1. Tim-1BKO mice were born at a Mendelian frequency. At 4-10 weeks of age they appeared normal and healthy by observation and histological examination of various organs/tissues, and did not display any obvious differences in terms of numbers and development of T and B cells, compared to control (WT, CD19Cre/WT, and CD19Cre/WTTim-1fl/WT, all of which were normal) mice (FIG. 1C). However, similar to Tim-1-/- B cells, Tim-1BKO B cells showed both reduced basal and induced IL-10 production (FIG. 1D), confirming the notion that Tim-1 defect impaired B cell IL-10 expression. As shown previously, compared to Tim-1- (negative) B cells, Tim-1+ B cells inhibited T cell proliferation and their IFN-γ and IL-17 production but increased IL-10 production in T/B cell cocultures. While B cells from Tim-1BKO mice, like those from mice with global Tim-1 defects, promoted T cell proliferation and their IFN-γ and IL-17 production, but inhibited IL-10 production, compared to wildtype (WT) B cells (FIG. 1E).

Thus, as shown herein, Tim-1BKO mice have successfully been generated. The major abnormality of the mice at early age, like gobally deficient Tim-1-/- and Tim-1Δmucin mice, is impaired B cell IL-10 production and suppressive function, Tim-1BKO mice with age develop spontaneous inflammation in multiple organs and tissues. Interestingly, Tim-1BKO mice along with age, like Tim-1-/- and Tim-1Δmucin mice, showed various abnormalities. Typically, at 8-10+ months of age, almost all of Tim-1BKO mice examined had enlarged spleens (and various lymph nodes) with 2-5 fold increased immune cells, which were predominantly CD11b+ cells and CD4+ and CD8+ T cells but with similar number of B cells (percentage decreased), compared to co-housed control (WT, CD19Cre/WT, and CD19Cre/WTTim-1fl/WT) mice (FIGS. 2A, 2B). Their T cells displayed more activated/memory-like phenotypes (CD44hi /CD62Llow /CD69hi /CD25hi ) and produced more IFN-y and IL-17, while frequency of Foxp3+ Tregs decreased (FIG. 2B). The old Tim-1BKO mice also showed increased activation of CD11c+ , CD11b+ , and especially B cells as they all had increased CD80/CD86/MHC II expression (FIG. 2C). Some of the mice showed weight loss and/or prolapse with enterocolitis (FIG. 2D) associated with increased infiltration of T cells as well as CD11b+ cells, including Gr1+ granulocytes. These T cells produced more IFN-γ and IL-17 but less IL-10 (FIG. 7). Some of the mice showed skin inflammation (FIG. 2D), typically in upper body, around face, neck and arms. Strikingly, unlike Tim-1-/- and Tim-1Δmucin mice, some of the mice also developed spontaneous EAE-like disease mainly with accumulation of CD11b+ (F4/80+ macrophage, Gr1+ granulocytes, and CD11c+ myeloid dendritic cells) cells as well as T cells in brains. Histological examination showed the mice developed meningeal granulomatous inflammation over brain with Touton giant cells. Histological examination also showed immune cell infiltrates in livers, kidneys, and several other organs/tissues. Collectively, these data strongly support that Tim-1 is required for Bregs-mediated tolerance maintenance, such that loss of Tim-1 in Bregs compromises their function and leads to break in self tolerance and development of inflammation in multiple organs/tissues, even in brain in the hosts, due to impaired regulatory mechanisms and increased inflammatory responses.

### Young Tim-1BKO mice develop more severe MOG35-55-induced EAE.

Since Tim-1BKO mice at early age were overall healthy, it was determined whether the young mice would develop more severe induced inflammatory disease, due to Tim-1 deficiency mediated impairment of Breg function. Indeed, upon immunization with MOG35-55, young Tim-1BKO mice developed a more severe EAE, which could not recover, compared to control mice (Fig. 3a). Cells from draining lymph nodes (dLNs) of the mice with EAE had 2-3 times the basal proliferation in the absence of any exogenous antigen. These cells also showed increased dose dependent proliferation upon addition of antigen, compared with those from control mice. Cytokine analyses showed that with or without antigenic restimulation, the dLN cells from immunized Tim-1BKO mice secreted more IFN- y and IL-17 (FIG. 3B), which correlated with their proliferation observed in cultures. Furthermore, the CNS of the immunized mice showed reduced frequency/number of Foxp3+ Tregs and IL-10+ T cells, increased frequency/number of IL17+ T cells, and increased number but not frequency of IFN- γ+ T cells. These data indicate that, in addition to being required for Breg-mediated tolerance maintenance, Tim-1 is also required for Bregs in suppressing inflammation by regulating the balance of pathogenic Th1/Th17 cells and regulatory Foxp3+ and IL-10+ regulatory T cells.

### Tim-1+ B cells highly express IL-10 as well as a set of co-inhibitory molecules and transcription factors (TFs).

In order to gain more insights into the role of Tim-1 in Bregs, Nanostring analysis was performed on Tim-1+ and Tim-1- (negative) B cells from naive WT and Tim-1Δmucin mice, using a custom nanostring codeset containing signature genes (e.g., IL-10) for both IL10-producing Tr1 cells and exhausted T cells. Tim-1Δmucin is a loss-of-function Tim-1 mutant, but mutant Tim-1 is still expressed on cell surface and positively stained by anti- Tim-1, thus Tim-1Δmucin mouse is a valuable tool for identifying Tim-1Δmucin+ cells and studying the effect of loss of Tim-1 signaling. These analyses demonstrate that, in addition to IL-10, Tim-1+ B cells also showed enriched expressed of genes that encode coinhibitory molecules (e.g., Tigit, Lag3, Ctla4, Pdcd1, which encodes PD 1, and Havcr2, which encodes TIM-3) and regulatory molecule fgl2, and TFs (Prdm1, which encodes Blimp-1, AhR, Irf4, and Id2) (FIG. 4A), all of which was confirmed by realtime PCR analysis. Enriched cell surface expression of TIGIT, LAG3, PD1, and Tim-3 in Tim-1+ B cells was further confirmed by flow cytometry (FIG. 4B). Interestingly, expression of all these genes, except Pdcd1, was reduced in Tim-1Δmucin+ cells (FIG. 4A), indicating their expression requires Tim-1 signaling. As Tim-1+ B cells highly express IL-10 as well as a set of co-inhibitory molecules and TFs, it raised the possibility that Tim1+ Bregs maintain self-tolerance and suppress inflammation by using multiple regulatory mechanisms, and Tim-1 expression and signaling is required for the regulatory function of Bregs by regulating the expression of many of these negative molecules via modulating expression of a unique set of TFs, as identified herein.

### Role of TIGIT in Bregs

TIGIT has been shown to be expressed mainly in NK, Foxp3+ Tregs and activated T cells and is important in suppressing immune responses. Since the data described herein demonstrate TIGIT expression in B cells and its enrichment in Tim-1+ Bregs (FIGS. 4A-4B), it was further examined to look at the role of co-inhibitory molecules in Bregs. Consistent with the requirement of Tim-1 signaling for TIGIT expression, Tim-1 ligation with an anti-Tim-1 mAb increased TIGIT expression as well as IL-10 production from B cells (FIG. 5A). Most TIGIT+ B cells are also IL-10+ , interestingly however, there is about 30% of TIGIT+ B cells which are IL-10- , raising the possibility, without wishing to be bound or limited by theory, that these TIGIT+ IL-10- cells may not require IL-10 for their regulatory function. Also, many of IL-10+ cells are TIGIT-, indicating their function may not need TIGIT, without wishing to be bound or limited by theory (FIG. 5A).

Next we studied whether TIGIT and IL-10 affect each other's expression. While blockade IL-10 with anti-IL-10 mAb did not affect basal or Tim-1 ligation-induced TIGIT expression in B cells (FIG. 5B), B cells from TIGIT-deficient (Tigit-/- ) mice produced less IL-10 (~30-40% decrease in all conditions) upon treatment with a number of inductive stimuli, indicating that TIGIT in B cells is required for optimal Breg IL-10 production (FIG. 5C).

As it has been previously shown that B cells with Tim-1 defects promoted Th1 and Th17 differentiation and inhibited iTreg and Tr1 cell generation, it was studied whether Tigit-/- B cells affect T cell differentiation. Indeed, Tigit-/- B cells promoted Th1 and Th17 differentiation, and inhibited iTregs and Tr1 generation (FIG. 5D), which is consistent with the recent finding that TIGIT in Tregs selectively inhibits Th1 and Th17 cell responses.

It was then examined whether Tigit-/- Tim-1+ Bregs affect the development and severity of EAE. As reported previously, WT Tim-1+ B cells strongly inhibited EAE severity, however, Tigit-/- Tim-1+ Bregs were not as effective as the WT Tim-1+ B cells in suppressing EAE upon adoptive transfer (FIG. 5E). To further demonstrate the role of TIGIT in Bregs, TIGIT floxed mice on C57BL/6 background were generated by introducing two Loxp sites flanking the exon 1 of the Tigit gene and crossed the mice with CD 19cre mice to obtain mice with TIGIT deficiency only in B cells (CD19Cre/WTTigitfl/fl or TigitBKO ). Efficient B cells TIGIT deletion was confirmed by both qPCR and flow cytometry (FIG. 8). TigitBKO mice within 8-10 weeks of age were normal without any notable defects in T and B cell development, except reduced IL-10 production in B cells. Strikingly however, some of the TigitBKO mice at as early as 4 months of age preferentially developed EAE-like symptom with paralysis with substantial infiltration of macrophage and granulocytes as well as T cells in the brain (FIG. 5F). Consistently, histological examination showed the mice developed meningeal granulomatous inflammation. However, histological examination did not show notable inflammation in other organs or tissues.T cells from these mice became activated and produed more IFN-y and IL-17, without notable change in Foxp3+ Tregs.

Furthermore, like young Tim-1BKO mice, young TigitBKO mice also developed a more severe MOG35-55-induced EAE, which could not recover, compared to control mice, associated with increased Th1 and Th17 cells and reduced Foxp3+ Tregs and Tr1 cells in the CNS.

Collectively, these data support that TIGIT, a coinhibitory molecule differentially expressed in Bregs and regulated by Tim-1 signaling, is not only critical for Bregs in suppressing inflammation by regulating the balance of pathogenic Th1/Th17 cells and Foxp3+ /IL-10+ regulatory T cells, it is also required for Breg-mediated tolerance maintenance, preferentially in brain.

### Role of AhR in Bregs

Tim-1+ B cells also preferentially express a set of TFs, some of which were regulated by Tim-1 signaling. Of them, both IRF4 and Blimp-1 have been shown to be involved in the regulation of IL-10+ Bregs. Interestingly, AhR is highly expressed in Tim-1+ B cells, but dramatically reduced Tim-1Δmucin+ cells, due to loss of Tim-1 signaling (FIGS. 4A-4B). Consistent with this, Cyp1a1, a direct AhR target is also higher in Tim-1+ B cells but is reduced to basal level in Tim-1Δmucin+ cells (FIG. 4B). AhR in several other types of immune cells has been shown to be critical in immunosuppression by regulating their regulatory function such as IL-10 production. Thus, its role was further examined in Bregs.

Consistent with the mRNA data, expression of AhR proteins was higher in Tim-1+ B cells than in Tim-1- B cells (FIG. 6A). Correlated with the production of IL-10, basal level of AhR proteins in Tim-1-/- B cells was ~40-50% lower than that in WT cells, and upon Tim-1 ligation, its expression dramatically increased in WT cells but did not increase obviously in Tim-1-/- cells (FIG. 6B). AhR in B cells bound to the XRE in the IL10 promoter, as previously shown in Tr1 cells, and this binding was increased upon Tim-1 ligation. Interestingly, AhR also bound to the XRE in the Tigit promoter in B cells which was also enhanced upon Tim-1 ligation. Furthermore, by using B cells from AhRd mice, which express a mutant form of AhR binding to its ligands with much lower affinity, it was found that the AhRd mutant not only impaired both basal level expression of IL-10 and TIGIT, it also completely abolished Tim-1 ligation-induced IL-10 production and largely reduced Tim-1 ligation-induced TIGIT expression (FIG. 6C). These data, together with the notion that Tim-1 defects in Bregs impair expression of AhR as well as IL-10 and TIGIT, indicate that Tim-1 regulates Breg IL10 and Tigit gene activation via modulating AhR expression.

Given that Tim-1 as a phosphatidylserine (PS) receptor is required for IL-10 production of Bregs by sensing apoptotic cells, and Tim-1 defects impair optimal IL-10 production in Bregs, and that inflammatory cytokines such as IL-21 and IL-1 plus IL-6 are critical in promoting IL-10+ Bregs, it was next compared how AhR, Tim-1 and AC affect Breg IL-10 production induced by these inflammatory cytokines, by using AhRd and Tim-1-/- B cells and by blocking AC binding to Tim-1 in B cells using PS-binding Annexin V. As Tim-1 is required for AC binding to B cells, addition of Annexin V had no obvious effect on IL-10 production in all the Tim-1-/- B cell cultures, while addition of Annexin V reduced WT B cell IL-10 to the levels of Tim-1-/- B cells in all conditions. Consistent with the requirement of AhR for Tim-1-mediated Breg IL-10 production, AhRd B cells showed a similar reduction of both basal and induced IL-10 production as compared to Tim-1-/- B cells, upon treatment with IL-21 or IL-1 plus IL-6, and addition of Annexin V also had no effect on IL-10 induction in AhRd B cells (FIG. 6E). Furthermore, it was found that IL-21 and IL-1 plus IL-6 also promoted TIGIT expression in B cells, which was impaired by AhRd mutant and Tim-1 deficiency. These data indicate that the AC/Tim-1/AhR axis is required for optimal IL-10 production and TIGIT expression of Bregs induced by inflammatory cytokines.

It was then examined how AhR defect in B cells affect EAE by using the B cell deficient muMT mice. Consistent with a previous report, muMT mice could not recover well once EAE developed, due to reduced Foxp3+ and IL-10+ regulatory T cells and increased Th1/Th17 responses, while transfer of WT B cells into muMT mice reduced EAE which is comparable to WT mice, due to restored balance between Foxp3+ /IL-10+ Tregs and increased Th1/Th17 responses. Interestingly however, AhRd B cells, similar to Tim-1-/- B cells strongly promoted EAE in the hosts which could not recover. Compared to muMT mice only, hosts with Ahrd B cells or Tim-1-/- B cells showed further increased Th1/Th17 responses and likely also further reduced Foxp3+ /IL-10+ Tregs (FIG. 6F). Collectively, these findings suggest that AhR, whose optimal induction and maintenance require Tim-1-mediated signaling, is critical and essential for AC/Tim-1 axis-mediated function of Bregs by directly activating their IL10 and Tigit genes. AC/Tim-1/AhR axis is also required for the development and optimal function of Bregs induced by inflammatory cytokines.

### Discussion

Most studies have examined the function of Bregs by transferring Bregs with various exclusive markers in induced inflammatory disease settings and support that Bregs are important in limiting inflammation. However, whether Bregs are important in maintaining self-tolerance in unmanipulated hosts has not been demonstrated. Described herein is evidence that in addition to being critical for restraining inflammation in induced disease settings, Bregs are also critical and essential in maintaining self tolerance at the steady state in unmanipulated mice.

It has been previously shown that Tim-1 as a phosphatidylserine receptor is required for optimal Breg IL-10 production by sensing apoptotic cells (AC), and mice with global Tim1 defects displayed impaired Breg IL-10 production and with age spontaneously developed inflammation in multiple organs and tissues. However, the role of Bregs in maintaining self-tolerance cannot be firmly concluded as Tim-1 deficient non-B cells may also contribute to the outcomes in these mice.

As described herein, mice with B cell specific Tim-1 deficiency (Tim-1BKO ) have been generated and it has been discovered that the mice developed spontaneous inflammation in multiple organs and tissues. The Tim-1BKO mice started to show some of the abnormalities at as early as about 8 months of age, which is faster than mice with global Tim-1 defects that typically showed abnormalities after 10 months of age. Also, one of the unique features of the Tim-1BKO mice is that some of the mice develop spontaneous EAE-like paralytic disease with inflammation in brain, which has not been observed in mice with global Tim-1 defects, indicating that Tim-1 deficient non- B cells overall negatively contribute to the outcomes in these mice. Development of spontaneous paralytic symptom in Tim-1BKO mice has not been observed hitherto with loss of any of the negative regulatory molecules such as IL-10, CTLA-4 or PD1. Importantly, since the Tim-1BKO mice are not on a CNS antigen-specific TCR or BCR background, this emphasizes that, in addition to its critical role in maintaining self-tolerance systemically, Bregs also have a unique role in maintaining the CNS tolerance and regulating CNS autoimmunity.

Bregs have been shown to restrain inflammatory responses by targeting multiple types of immune cells. Consistent with the notion that Bregs are critical in maintaining/inducing Foxp3+ Tregs and Tr1 cells and suppressing inflammatory effector T cells, Tim-1BKO mice with impaired Breg function displayed increased IFN-γ and IL-17 production in T cells with more activated phenotypes and decreased Foxp3+ Tregs and Tr1 cells, not only along with age at the steady state, but also during induced inflammation at young age. Tim-1BKO mice with age also displayed increased activation of APCs, indicating that Bregs are also required for inhibiting APC function. Especially, B cells in Tim-1BKO mice with age showed substantial activation, indicating that Bregs are most likely critical in directly suppressing "non-Breg" B cells. Also, since B cell deficient mice have not been reported to develop spontaneous inflammation, although the mice not only lack Bregs but also have reduced Foxp3+ Tregs, this strongly argures that the "non-Breg" B cells are most likely required for the development of spontaneous inflammation in Tim1BKO mice. In this regard, as shown herein, B cells with Tim-1 defects were better APCs and produced more proinflammatory cytokines in regulating the balance between proinflammatory and regulatory T cells towards a dominant proinflammatory T cell response. Another interesting feature in Tim-1BKO mice is the accumulation of CD11b+ macrophages, and especially granulocytes, with age in peripheral lymphoid compartments and various organs and tissues such as brain and gut. Particularly, the mice with EAE-like paralytic symptom displayed meningeal granulomatous inflammation over brain with Touton giant cells. It is possible that Bregs also directly inhibit the activation and accumulation of these cells. However, it may be also likely that Bregs regulate these cells indirectly through modulating other types of immune cells, such as T cells.

The fact that Tim-1 is expressed only in less than 10% of B cells, but Tim-1BKO mice developed both spontaneous and more severe induced inflammatory diseases emphasizes the important role of Tim-1, serving as a PS receptor by sensing AC, in the development, maintenance, and function of Bregs. Most studies in various inflammatory settings have shown that Bregs suppress inflammation primary via the production of IL10. However, IL-10-independent mechanisms for Breg-mediated suppression are also being increasingly recognized. Indeed, as described herein, it was found that, in addition to IL-10, Bregs also differentially express a set of coinhibitory molecules such as TIGIT. Importantly, Tim-1 expression and signaling are required for optimal expression of many of the molecules. Interestingly, all the inhibitory molecules are also expressed in Foxp3+ Tregs, which enable specialized regulatory functions of Foxp3+ Treg subsets. This seems also true for Bregs. Although Tim-1BKO mice displayed impaired Breg IL-10 production, development of spontaneous inflammation in multiple organs and tissues in the mice apparently cannot be attributed to impaired IL-10 production in Bregs, as mice with B cell-specific IL-10 deficiency have been reported not to develop spontaneous inflammation along with age. Instead, it was found that mice with B cell-specific TIGIT deficiency (TigitBKO ) developed spontaneous inflammation. However, different from Tim-1BKO mice which developed spontaneous inflammation in multiple organs and tissues, including brain, TigitBKO mice preferentially developed spontaneous EAE-like paralytic symptom without notable inflammation in other organs or tissues. On the other hand, upon EAE induction with MOG35-55, Tim-1BKO mice, TigitBKO mice and mice with B cell-specific IL-10 deficiency all had Breg IL-10 defects and developed more severe induced EAE without recovery.

These data indicate that Bregs regulate tolerance and inflammation through diverse regulatory mechanisms, and individual regulatory mechanism can operate in a particular tissue and inflammatory setting by regulating certain type(s) of immune cells and immune responses. In unmanipulated mice, TIGIT is required for Bregs in maintaining self-tolerance preferentially in brain, however, there must be other regulatory mechanisms (preferentially or coordinately) responsible for Breg-mediated self-tolerance in other organs and tissues. While in inflammatory settings, such as induced EAE, IL-10 is absolutely required for Breg-mediated suppression of inflammation. Since TIGIT is required for optimal Breg IL-10 production, while IL-10 seems not involved in TIGIT expression in Bregs, this data indicates that one regulatory mechanism can differentially regulate other regulatory mechanisms in Bregs, and thus can enhance specialized regulatory functions of Bregs.

In addition to IRF4 and Blimp-1, which have been shown to regulate IL-10+ Bregs during inflammation, it was also identified AhR is preferentially expressed in Bregs and demonstrated that AhR is required for Breg-mediated suppression of induced CNS inflammation and allograft rejection. Maintenance and induction of AhR in Bregs require Tim-1 expression and signaling, and AhR is almost entirely required for AC/Tim-1 axis-mediated expression of IL-10 and TIGIT in Bregs by directly binding and activating the genes. It is possible that AhR in Bregs also regulates other regulatory mechanisms, in addition to IL-10 and TIGIT. In this regard, the computational analysis described herein has identified putative AhR-binding sites in the promoter regions of other coinhibitory molecules, such as Tim-3 and LAG3. Thus, it is very likely, without wishing to be bound or limited by theory, that AhR can serve as a critical and essential TF directly regulating a set of negative regulators by binding to their promoters in Bregs, and AhR defect in Bregs can impair expression of these regulators. Since AhR-deficient and Tim-1-deficient B cells displayed comparable regulation of immune responses and development of the CNS inflammation, it indicates that AhR may be mainly required in regulating AC/Tim-1-mediated Breg-associated gene expression and function. However, as AhR defect does not fully abrogate IL-10 and TIGIT expression, it indicates that there are also other TFs (e.g., IRF4, Blimp-1 or Id2) responsible for the optimal expression of these molecules.

It has been previously shown that AC/Tim-1 axis is required for Breg optimal IL-10 production induced by signaling via BCR, CD40 and TLR, which are all have been shown to involve in regulating IL-10+ Bregs. Recent studies have providence that during inflammation, proinflammatory cytokines such as IL-21, IL-1 and IL-6 are critical in the promotion of Breg IL-10 production. Interestingly, defects in the AC/Tim-1/AhR pathway also impair both these proinflammatory cytokine-promoted IL-10 production and TIGIT expression in Bregs, further support the importance and requirement of the AC/Tim-1 axis in the development, maintenance, and optimal function of Bregs both at the steady state and during inflammation.

In summary, as demonstrated herei, Bregs are essential in maintaining self-tolerance and restraining inflammation, in which AC/Tim-1/AhR axis is required for optimal Breg function by regulating multiple regulatory mechanisms. Bregs regulate self-tolerance and inflammation by using different regulatory mechanisms, which may differentially and/or coordinately operate at particular organs and tissues by targeting certain inflammatory responses at different inflammatory settings. In addition to their importance in self-tolerance and autoimmunity, Bregs are also critical in cancers, infections, and transplantation.

### Materials and Methods

### Mice and Reagents

C57BL/6 mice, muMT, and Ahrd mice were purchased from The Jackson Laboratory. Tim-1-/- and Tim-1Δmucin mice have been described. MOG35-55 was synthesized by Quality Controlled Biochemicals. Cytokines and antibodies for cell culture, flow cytometry, and cytometric bead array were obtained from BioLegend, eBioscience, BD Biosciences, and R&D Systems. Anti-Tim-1 antibody RMT1-4 (BioLegend) was used for flow cytometry. Anti-Tim1 antibody 5F12 was described previously.

### Cell Purification and Cultures

Splenic CD19+ B cells from 2-4 month old mice were purified using MACS columns following staining with anti-mouse CD19 MACS beads. Cells were cultured in roundbottom 96-well plates in the presence of anti-Tim-1 (clone 5F12), (Fab')2 fragment anti-IgM, Anti-CD40, IL-21, or their combinations. After 3 days, IL-10 production in culture supernatants was measured by cytokine bead array (CBA) or ELISA. MACS purified CD19+ B cells were labeled with PE-anti-Tim-1 (RMT1-4) and then separated into Tim-1+ and Tim-1- B cells by fluorescence-activated cell sorting for further uses. CD4+ CD62Lhi CD25- naive CD4+ T cells were purified by fluorescence-activated cell sorting after a MACS bead isolation of CD4+ cells as previously described.

Naive CD4+ cells were activated with either plate-bound anti-CD3 (2 µg/ml) and anti-CD28 (2 µg/ml) or B cells plus soluble anti-CD3 (1 µg/ml) under Th0 (no cytokine), Th1 (IL-12 + anti-IL-4), Th2 (IL-4 + anti-IL-12/anti-IFN-g), Th17 (TGF-b1 + IL-6), Tr1 (TGF-b1 + IL-27), and iTreg (TGF-b1) conditions. After 96 h, cells were collected for further experiments.

For isolation of CNS-infiltrating mononuclear cells, mice were first perfused through the left cardiac ventricle with cold PBS. The forebrain and cerebellum were dissected and spinal cords flushed out with PBS by hydrostatic pressure. CNS tissue was cut into pieces and digested with collagenase D (2.5 mg/ml, Roche Diagnostics) and DNase I (1 mg/ml, Sigma) at 37 °C for 30 min. Mononuclear cells were isolated by passing the tissue through a 70 mm cell strainer, followed by a 70%/37% percoll gradient centrifugation. Mononuclear cells were removed from the interphase, washed, and resuspended in culture medium for further analysis.

### Flow Cytometry

For intracellular cytokine staining, cells were stimulated in culture medium containing phorbol 12-myristate 13-acetate (30 ng/ml, Sigma-Aldrich), ionomycin (500 ng/ml, Sigma-Aldrich), and GOLGISTOP (1 µl/ml, BD Biosciences) in a cell incubator with 10% CO2 at 37°C for 4 h. After surface markers were stained, cells were fixed and permeabilized with CYTOFIX/CYTOPERM and Perm/Wash buffer (BD Biosciences) according to the manufacturer's instructions. Then, cells were stained with fluorescenceconjugated cytokine Abs at 25°C for 30 min before analysis. 7-AAD (BD Biosciences) was also included to gate out the dead cells. All data were collected on a FACSCALIBUR or an LSR II (BD Biosciences) and analyzed with FLOWJO software (TreeStar). EAE and Heart Transplant

CD19+ B cells were transferred into muMT mice. Mice were immunized subcutaneously in the flanks with an emulsion containing MOG35-55 (100 µg/mouse) and M. tuberculosis H37Ra extract (3 mg/ml, Difco Laboratories) in CFA (100 µl/mouse). Pertussis toxin (100 ng/mouse, List Biological Laboratories) was administered intraperitoneally on days 0 and 2. Mice were monitored and assigned grades for clinical signs of EAE as previously described.

### Analysis of gene expression by NANOSTRING

Total RNA isolated from purified Tim-1+ and Tim-1- B cellsRNA cells were hybridized with a custom-made Code Set containing the signature genes of Tr1 and exhausted T cells. Barcodes were counted (1,150 fields of view per sample) on an nCounter Digital Analyzer following the manufacturer's protocol (NANOSTRING Technologies Inc.). Data were processed first by normalization with respect to the geometric mean of the positive control spike counts (provided by the manufacturer) and then with 4 reference genes (Actb, Gapdh, Hprt, and Tubb5). A background correction was done by subtracting the mean plus 2 SDs of the 8 negative control counts (provided by the manufacturer) and eliminating data that were less than 1.

### RNA isolation and Real-time PCR

RNA was extracted with RNEASY Plus kits (Qiagen) and cDNA was made by ISCRIPT(BioRad). All of the real-time PCR probes were purchased from Applied Biosystems. Quantitative PCR were performed using VIIA^{™} 7 Real-Time PCR System (Applied Biosystems). Statistics

The clinical score and incidence of EAE were analyzed by Fisher's exact test, and comparisons for CBA and real-time PCR results were analyzed by Student's t test. P < 0.05 was considered significant.

## Claims

1. A therapeutic composition comprising a multispecific binding agent comprising a moiety that binds and inhibits the activity of the B-cell regulator TIGIT and a moiety that binds a B-cell-specific cell-surface polypeptide marker selected from CD19, CD20, and CD22.

2. The therapeutic composition of claim 1, wherein the moiety that binds and inhibits the activity of the B-cell regulator comprises an antigen-binding domain of an antibody that specifically binds the B-cell regulator.

3. The therapeutic composition of any one of claims 1 or 2, wherein the moiety that binds a B-cell-specific cell-surface polypeptide marker comprises an antigen-binding domain of an antibody that specifically binds a B-cell-specific cell surface marker.

4. The therapeutic composition of any one of claims 1-3, wherein the antigen-binding domain is comprised by an scFV or a nanobody.

5. The therapeutic composition of any one of claims 1-4 for use in the treatment of cancer or a chronic infection.

6. An inhibitor of TIGIT for use for treating cancer or a chronic infection, wherein the inhibitor of TIGIT comprises a binding moiety that binds to TIGIT, and is specifically targeted to B-cells via a binding moiety for a B-cell cell-surface marker selected from CD19, CD20 and CD22.

7. The inhibitor of TIGIT for use of claim 6, wherein the inhibitor of TIGIT comprises a binding moiety that binds to TIGIT, and comprises a multispecific binding agent comprising a moiety that binds and inhibits the activity of TIGIT, and a binding moiety for a B-cell-specific cell-surface polypeptide marker selected from CD19, CD20 and CD22.

8. The inhibitor of TIGIT for use of claim 6 or claim 7, wherein the binding moiety that binds and inhibits the activity of TIGIT comprises an antigen-binding domain of an antibody that specifically binds TIGIT.

9. The inhibitor of TIGIT for use of any one of claims 6-8, wherein the binding moiety that binds a B-cell-specific cell-surface polypeptide marker comprises an antigen-binding domain of an antibody that specifically binds a B-cell-specific cell surface marker.

10. The inhibitor of TIGIT for use of claim 9, wherein the B-cell-specific cell surface marker is selected from CD19, CD20, and CD22.

## Patentansprüche

1. Therapeutische Zusammensetzung, die ein multispezifisches Bindemittel umfasst, umfassend eine Einheit, die den B-Zell-Regulator TIGIT bindet und seine Aktivität hemmt, und eine Einheit, die einen B-Zell-spezifischen Zelloberflächen-Polypeptidmarker bindet, ausgewählt aus CD19, CD20 und CD22.

2. Therapeutische Zusammensetzung nach Anspruch 1, wobei die Einheit, die den B-Zell-Regulator bindet und seine Aktivität hemmt, eine Antigenbindungsdomäne eines Antikörpers umfasst, der spezifisch den B-Zell-Regulator bindet.

3. Therapeutische Zusammensetzung nach einem der Ansprüche 1 oder 2, wobei die Einheit, die einen B-Zell-spezifischen Zelloberflächen-Polypeptidmarker bindet, eine Antigenbindungsdomäne eines Antikörpers umfasst, der spezifisch einen B-Zell-spezifischen Zelloberflächenmarker bindet.

4. Therapeutische Zusammensetzung nach einem der Ansprüche 1 bis 3, wobei die Antigenbindungsdomäne durch einen scFV oder einen Nanobody umfasst ist.

5. Therapeutische Zusammensetzung nach einem der Ansprüche 1 bis 4 zur Verwendung bei der Behandlung von Krebs oder einer chronischen Infektion.

6. Inhibitor von TIGIT zur Verwendung beim Behandeln von Krebs oder einer chronischen Infektion, wobei der Inhibitor von TIGIT eine Bindungseinheit umfasst, die an TIGIT bindet und über eine Bindungseinheit für einen B-ZellOberflächenmarker, ausgewählt aus CD19, CD20 und CD22, spezifisch auf B-Zellen ausgerichtet ist.

7. Inhibitor von TIGIT zur Verwendung nach Anspruch 6, wobei der Inhibitor von TIGIT eine Bindungseinheit umfasst, die an TIGIT bindet, und ein multispezifisches Bindemittel, das eine Einheit umfasst, die TIGIT bindet und dessen Aktivität hemmt, und eine Bindungseinheit für einen B-Zell-spezifischen Zelloberflächen-Polypeptidmarker, ausgewählt aus CD19, CD20 und CD22, umfasst.

8. Inhibitor von TIGIT zur Verwendung nach Anspruch 6 oder Anspruch 7, wobei die Bindungseinheit, die TIGIT bindet und dessen Aktivität hemmt, eine Antigenbindungsdomäne eines Antikörpers umfasst, der spezifisch TIGIT bindet.

9. Inhibitor von TIGIT zur Verwendung nach einem der Ansprüche 6 bis 8, wobei die Bindungseinheit, die einen B-Zell-spezifischen Zelloberflächen-Polypeptidmarker bindet, eine Antigenbindungsdomäne eines Antikörpers umfasst, der spezifisch einen B-Zell-spezifischen Zelloberflächenmarker bindet.

10. Inhibitor von TIGIT zur Verwendung nach Anspruch 9, wobei der B-Zellspezifische Zelloberflächenmarker aus CD19, CD20 und CD22 ausgewählt ist.

## Revendications

1. Composition thérapeutique comprenant un agent de liaison multispécifique comprenant un fragment qui se lie à TIGIT, un régulateur des lymphocytes B, et inhibe son activité et un fragment qui se lie à un marqueur polypeptidique de surface cellulaire spécifique des lymphocytes B choisi parmi CD19, CD20 et CD22.

2. Composition thérapeutique selon la revendication 1, dans laquelle le fragment qui se lie au régulateur des lymphocytes B et inhibe son activité comprend un domaine de liaison à l'antigène d'un anticorps qui se lie spécifiquement au régulateur des lymphocytes B.

3. Composition thérapeutique selon l'une quelconque des revendications 1 ou 2, dans laquelle le fragment qui se lie à un marqueur polypeptidique de surface cellulaire spécifique des lymphocytes B comprend un domaine de liaison à l'antigène d'un anticorps qui se lie spécifiquement à un marqueur de surface cellulaire spécifique des lymphocytes B.

4. Composition thérapeutique selon l'une quelconque des revendications 1 à 3, dans laquelle le domaine de liaison à l'antigène est compris dans un scFV ou un nanocorps.

5. Composition thérapeutique selon l'une quelconque des revendications 1 à 4 pour une utilisation dans le traitement du cancer ou d'une infection chronique.

6. Inhibiteur de TIGIT pour une utilisation pour traiter le cancer ou une infection chronique, dans lequel l'inhibiteur de TIGIT comprend un fragment de liaison qui se lie à TIGIT et qui est spécifiquement ciblé sur les lymphocytes B par l'intermédiaire d'un fragment de liaison pour un marqueur de surface cellulaire des lymphocytes B choisi parmi CD19, CD20 et CD22.

7. Inhibiteur de TIGIT pour une utilisation selon la revendication 6, dans lequel l'inhibiteur de TIGIT comprend un fragment de liaison qui se lie à TIGIT, et comprend un agent de liaison multispécifique comprenant un fragment qui se lie et inhibe l'activité de TIGIT, et un fragment de liaison pour un marqueur polypeptidique de surface cellulaire spécifique des lymphocytes B choisi parmi CD19, CD20 et CD22.

8. Inhibiteur de TIGIT pour une utilisation selon la revendication 6 ou la revendication 7, dans lequel le fragment de liaison qui se lie à TIGIT et inhibe son activité comprend un domaine de liaison à l'antigène d'un anticorps qui se lie spécifiquement à TIGIT.

9. Inhibiteur de TIGIT pour une utilisation selon l'une quelconque des revendications 6 à 8, dans lequel le fragment de liaison qui lie un marqueur polypeptidique de surface cellulaire spécifique des lymphocytes B comprend un domaine de liaison à l'antigène d'un anticorps qui se lie spécifiquement à un marqueur de surface cellulaire spécifique des lymphocytes B.

10. Inhibiteur de TIGIT pour une utilisation selon la revendication 9, dans lequel le marqueur de surface cellulaire spécifique des lymphocytes B est choisi parmi CD19, CD20 et CD22.
